# FASCICULE DE BREVET EUROPEEN

(11) **EP 3 065 732 B1**
(45) Date de publication et mention de la délivrance du brevet: **11.04.2018**
(21) Numéro de dépôt: 14781242.4
(22) Date de dépôt: 08.10.2014
(51) Int. Cl.: A61K 31/231, A23L 33/00, A61P 3/00, A61P 3/06, A61P 3/10, A61K 31/201, A61P 11/00, A61P 11/12

(54) **COMPOSES, COMPOSITIONS ET UTILISATIONS CORRESPONDANTES, POUR LA PREVENTION ET/OU LE TRAITEMENT DES DYSLIPIDEMIES**
VERBINDUNGEN, ZUSAMMENSETZUNGEN UND VERWENDUNGEN DAVON ZUR PRÄVENTION UND/ODER BEHANDLUNG VON DYSLIPIDÄMIE
COMPOUNDS, COMPOSITIONS AND USES THEREOF FOR THE PREVENTION AND/OR TREATMENT OF DYSLIPIDEMIA

(30) Priorité: 08.10.2013 FR 1302334
(43) Date de publication de la demande: 14.09.2016
(73) Titulaire: Centre National de la Recherche Scientifique (C.N.R.S.), 75016 Paris (FR); Université De Poitiers, 86034 Poitiers Cedex (FR)
(72) Inventeur: FERREIRA, Thierry, F-86240 Iteuil (FR); FERRU-CLEMENT, Romain, F-86000 Poitiers (FR); VANDEBROUCK, Clarisse, F-86000 Poitiers (FR)
(74) Mandataire: Vidon Brevets & Stratégie
(86) Numéro de dépôt international: PCT/EP2014/071543
(87) Numéro de publication internationale: WO 2015/052237

(56) Documents cités:
- EP-A2- 0 104 043
- WO-A1-2010/149170
- WO-A2-02/083059
- WO-A2-2012/054527
- STONE VIRGINIA M ET AL: "The cytoprotective effects of oleoylethanolamide in insulin-secreting cells do not require activation of GPR119.", BRITISH JOURNAL OF PHARMACOLOGY, vol. 165, no. 8, avril 2012 (2012-04), pages 2758-2770, XP002721326, ISSN: 1476-5381
- MORGAN N G ET AL: "Unsaturated fatty acids as cytoprotective agents in the pancreatic <2>-cell", PROSTAGLANDINS LEUKOTRIENES AND ESSENTIAL FATTY ACIDS, vol. 82, no. 4-6, 1 avril 2010 (2010-04-01), pages 231-236, XP027022275, CHURCHILL LIVINGSTONE, EDINBURGH ISSN: 0952-3278, DOI: 10.1016/j.plefa.2010.02.018
- PINEAU LUDOVIC ET AL: "Lipid-induced ER stress: synergistic effects of sterols and saturated fatty acids.", TRAFFIC (COPENHAGEN, DENMARK), vol. 10, no. 6, juin 2009 (2009-06), pages 673-690, XP002733951, ISSN: 1600-0854 cité dans la demande
- LAURIE-ANNE PAYET ET AL: "Saturated Fatty Acids Alter the Late Secretory Pathway by Modulating Membrane Properties", TRAFFIC, vol. 14, no. 12, 2 décembre 2013 (2013-12-02), pages 1228-1241, XP055159381, ISSN: 1398-9219, DOI: 10.1111/tra.12117
- PAYET LAURIE-ANNE ET AL: "Cystic fibrosis bronchial epithelial cells are lipointoxicated by membrane palmitate accumulation.", PLOS ONE, vol. 9, no. 2, E89044, 2014, pages 1-12, XP002733952, ISSN: 1932-6203, DOI: 10.1093/glycob/cwt056
- Laurie-Anne Payet: "Effets des acides gras saturés sur la voie de sécrétion. Relation avec la mucoviscidose", Thèse Université de Poitiers , 29 novembre 2013 (2013-11-29), XP055159587, Extrait de l'Internet: URL:http://nuxeo.edel.univ-poitiers.fr/nux eo/site/esupversions/e25b5dd2-e52f-4642-a6 45-072e90abd59d [extrait le 2014-12-18]

## Description

### 1. Le domaine de l'invention

La présente invention concerne le domaine de la médecine. Elle concerne plus particulièrement l'utilisation de composés pour prévenir et/ou traiter, chez un sujet, une lipointoxication, notamment une lipointoxication par hypoxie. La lipointoxication, ou dyslipidémie dans son acception large, est typiquement liée à la présence en excès dans les membranes biologiques, y compris dans les membranes biologiques de cellules non adipocytaires, d'acides gras, en particulier d'acides gras à longues chaînes saturées, et/ou de stérols. L'invention concerne également des compositions, en particulier les compositions pharmaceutiques et suppléments ou compléments alimentaires, comprenant de tels composés, pour leurs utilisations pour prévenir et/ou traiter une lipointoxication par hypoxie. Les composés et compositions selon l'invention peuvent en particulier être avantageusement utilisés pour prévenir et/ou traiter une pathologie choisie parmi les pathologies pulmonaires, notamment la mucoviscidose ou les broncho-pneumopathies chroniques obstructives.

### 2. Solutions de l'art antérieur

La mucoviscidose est une maladie affectant notamment les épithéliums glandulaires de nombreux organes. Cette maladie génétique létale à transmission autosomique récessive est liée à des mutations du gène CFTR (*cystic fibrosis transmembrane conductance regulator*) situé sur le chromosome 7, entraînant une altération de la protéine CFTR. Plus de 1900 mutations différentes ont déjà été identifiées. La mutation la plus fréquente est la mutation « F508del-CFTR » (ΔF508) qui consiste en une délétion de trois nucléotides au niveau du dixième exon du gène, aboutissant à l'élimination d'un acide aminé, la phénylalanine, en position 508. Le dysfonctionnement de la protéine CFTR provoque notamment une augmentation de la viscosité du mucus et son accumulation dans les voies respiratoires et digestives. D'un point de vue clinique, la forme la plus fréquente associe troubles respiratoires, troubles digestifs et troubles de la croissance staturopondérale. Les troubles pulmonaires restent la cause majeure de la morbidité et de la mortalité. La mucoviscidose entraine une inflammation chronique des bronches avec surinfection bactérienne, entraînant une dégradation progressive de l'état respiratoire, évoluant par poussées, débutant par des symptômes tels qu'une toux et aboutissant à une insuffisance respiratoire sévère. Divers traitements palliatifs symptomatiques - tels que la kinésithérapie, l'antibiothérapie, l'administration de médicaments mucolytiques - sont disponibles. A l'inverse, il n'existe à ce jour aucun traitement curatif, de nature médicamenteuse ou par un protocole de thérapie génique. Seul le médicament Ivacaftor®, comprenant une molécule potentiatrice de la protéine CFTR chez les sujets mucoviscidosiques porteurs de la mutation G551D, est actuellement commercialisé. Par ailleurs, plusieurs études pharmacologiques et/ou cliniques concernant des molécules d'intérêt thérapeutique ont été reportées. Ces molécules candidates visent généralement à corriger, réguler, potentialiser et/ou « chaperonner » la protéine CFTR dysfonctionnelle (cf. par exemple Kirby *et al.,* 2014 ; Pedemonte *et al.,* 2005 ; Van Goor *et al.,* 2006 ; Wang *et al.,* 2006; Sampson *et al.,* 2011 ; Van Goor *et al.,* 2011). Certaines ont notamment pour vocation de corriger le défaut de repliement de la protéine F508del-CFTR, qui l'empêche d'acquérir une conformation tridimensionnelle correcte, de progresser le long de la voie de sécrétion et d'accéder à son site de destination, la membrane plasmique. Cependant, la grande majorité des molécules candidates souffrent d'un manque d'efficacité dans les études *in vivo* et/ou les essais cliniques.

Les broncho-pneumopathies chroniques obstructives (BPCO) regroupent des maladies chroniques systémiques d'origine respiratoire et atteignant les bronches. La cause principale de cette maladie est le tabagisme. La BPCO se caractérise notamment par une obstruction lente et progressive des voies aériennes et des poumons, associée à une distension permanente des alvéoles pulmonaires. D'un point de vue clinique, les BPCO sont associées à des troubles respiratoires, avec de possibles complications neurologiques, cardiovasculaires ou musculaires. Les BPCO entraînent notamment une insuffisance respiratoire, pouvant être sévère. Divers traitements palliatifs symptomatiques - tels que l'antibiothérapie, la corticothérapie, l'utilisation d'un bronchodilatateur ou d'un support ventilatoire mécanique, voire l'oxygénothérapie de longue durée pour les formes les plus sévères - sont disponibles.

De récents travaux ont démontré que des cellules épithéliales bronchiques fraichement dissociées à partir de biopsies de patients mucoviscidosiques présentaient une accumulation anormalement élevée d'acide palmitique (AGS) au sein des phosphatidylcholines (PC) des membranes cellulaires (cf. Payet *et al.,* 2014). Préalablement, il avait été établi que la conversion des AGS cellulaires endogènes en AGI est catalysée par des enzymes dépendant de l'oxygène (Pineau *et al.,* 2008 ; Pineau *et al.,* 2009). Aussi, après avoir mis en évidence que l'hypoxie était induite dans des biopsies de patients mucoviscidosiques ou, à l'inverse, que l'hypoxie entrainait la lipointoxication de cellules épithéliales bronchiques, *in vitro* (cf. Payet *et al.,* 2014), il a été conclu que les cellules épithéliales bronchiques de patients mucoviscidosiques étaient lipointoxiquées par une accumulation d'acide palmitique causée par hypoxie. Par ailleurs, les inventeurs ont également pu réaliser le même type d'observations, reliant l'hypoxie à la lipointoxication, sur des biopsies pseudo-saines de patients fumeurs atteints d'un cancer du poumon (prélevées à l'extérieur de la zone pulmonaire cancéreuse).

Dans son acception large, une dyslipidémie - ou lipointoxication - se définit notamment comme une concentration anormalement élevée ou diminuée de lipides, typiquement d'acides gras libres ou non estérifiés, stérols (par exemple cholestérol, triglycérides ou phospholipides dans le sang). Les dyslipidémies se définissent alors comme une dérégulation de l'homéostasie lipidique

Les acides gras non estérifiés (AGNE) ou acides gras libres (AGL) représentent un élément énergétique important de l'organisme. Ils sont constitués d'un mélange complexe d'acides gras différant par leur nombre de double liaison et le nombre d'atomes de carbones constituant leur chaîne hydrocarbonée. D'origine endogène, ils sont formés par biosynthèse dans le cytoplasme des cellules et sont utilisés, sous forme d'acylcoA, pour la synthèse des triglycérides dans le tissu adipeux et le foie, notamment.

Dans le plasma, on retrouve principalement quatre acides gras qui représentent 85% des AGNE : acide oléique, palmitique, linoléique et stéarique. La majorité des AGNE est liée à l'albumine. Ils proviennent des triglycérides du tissu adipeux hydrolysés au cours du jeûne sous l'action de la lipoprotéine lipase tissulaire et sanguine en glycérol et acides gras. Leur concentration varie dans des proportions importantes en fonction de l'âge, de la prise de repas et de l'exercice physique. Généralement, en période post-prandriale, leur libération est supprimée.

Un stérol est un lipide possédant un noyau de stérane dont le carbone 3 est porteur d'un groupe hydroxyle. Les stérols sont considérés comme une sous-classe des stéroïdes. Le cholestérol, l'un des stérols les plus communs et répandus, est vital pour le fonctionnement cellulaire et est un précurseur de vitamines et d'hormones stéroïdiennes liposolubles.

Typiquement, dans son acception large, une dyslipidémie, au niveau cellulaire, correspond à une concentration anormalement élevée de lipides dans les membranes biologiques, et notamment à une accumulation d'acides gras saturés (AGS) dans les membranes biologiques. On parle également de dyslipidémie cellulaire. Une telle accumulation d'AGS dans les membranes biologiques conduit à la perturbation globale de la plasticité membranaire au niveau cellulaire. Ces phénomènes sont dénommés lipointoxications. Les lipointoxications installées sont responsables d'une perturbation de l'ensemble des mécanismes membranaires (détectable à toutes les étapes de la voie de sécrétion des protéines). Chez l'homme, à l'exception des adipocytes (seuls capables de synthétiser des lipides neutres et de les stocker), l'ensemble des types cellulaires est ainsi susceptible d'être concerné par la lipointoxication.

Jusqu'à présent, seuls des acides gras insaturés (AGI), en particulier l'acide oléique (huile d'olive), étaient connus pour contrer les effets délétères d'une intoxication liée à l'accumulation d'AGS (Cunha *et al.,* 2008; Diakogiannaki *et al.,* 2008; Katsoulieris *et al.,* 2009; Pineau *et al.,* 2009; Stein *et al.,* 1997; Wei *et al.,* 2006; Deguil *et al.,* 2011). Cependant, leur utilisation en tant qu'alicament et/ou médicament rencontre deux limites majeures. D'une part, les AGI présentent essentiellement des propriétés préventives et ont donc un intérêt limité dans le cadre du traitement des lipointoxications installées, i.e. des lipointoxications responsables d'une perturbation de l'ensemble des mécanismes membranaires (détectable à toutes les étapes de la voie de sécrétion des protéines). En effet, les AGI agissent en entrant en compétition directe avec les AGS, lors de la prise alimentaire, dans la synthèse des phospholipides (PL) membranaires. D'autre part, la toxicité des AGI a été démontrée sur les cellules incapables de transformer (« tamponner ») puis de stocker l'excès d'acides gras libres, notamment d'AGI, en lipides neutres, typiquement en triglycérides (TG) et/ou stérols estérifiés. C'est le cas par exemple pour une souche de levure chez laquelle, du fait de l'absence des quatre enzymes acyltransferases Lro1p, Dga1p, Are1p et Are2p, une dérégulation de la synthèse des lipides neutres est observée. Lors d'une exposition de cette souche mutante à une source d'AGI exogènes, la dérégulation lipidique se traduit par une prolifération massive des membranes intracellulaires puis par la mort des cellules, par un processus indépendant de l'UPR (Unfolded Protein Response ; voir ci-après) (Kohlwein & Petschnigg, 2007; Petschnigg *et al.,* 2011). De manière intéressante, des phénomènes identiques ont pu être observés dans des cellules de mammifères (Listenberger et al., 2003). Ceci explique pourquoi des acides gras insaturés deviennent toxiques pour la cellule dans des conditions de lipo-intoxication préalable de cette dernière, état dans lequel la capacité de stockage des acides gras insaturés sous la forme de lipides neutres, par la cellule, est dépassée (cellule lipo-intoxiquée qualifiée de « métaboliquement inactive »), ou, alternativement, dans des conditions normales, pour des cellules présentant une très faible capacité de synthèse de TG, telles que les cellules pancréatiques non-beta (Cnop Met al., 2001). Chez l'homme, à l'exception des adipocytes (seuls capables de synthétiser des lipides neutres et de les stocker), l'ensemble des types cellulaires est ainsi susceptible d'être concerné par la lipointoxication. Il est notamment connu que les perturbations liées à l'accumulation d'AGS conduisent à l'apoptose des cellules β-pancréatiques responsables de la synthèse d'insuline (Butler *et al.,* 2003) ou à celle des hépatocytes (Egnatchik et al., 2014).

Ainsi qu'évoqué précédemment, aucune stratégie thérapeutique actuelle ne vise à restaurer, à la base, la fonctionnalité des cellules et organes lipointoxiqués et n'est donc en mesure d'intervenir au niveau d'étapes précoces dans la cascade des effets délétères rencontrés dans les pathologies pulmonaires entraînant notamment une insuffisance respiratoire, typiquement en amont de chacune des étapes ciblées par les traitements existants. De plus, les travaux des inventeurs indiquent que la non prise en compte du contexte de lipointoxication, dans le cadre des pathologies pulmonaires notamment, et de la mucoviscidose en particulier, pourrait être responsable d'un verrou technologique et rendre compte de l'absence de traitement curatif, jusqu'à présent.

Les inventeurs décrivent à présent des molécules ou composés, et compositions comprenant de tels molécules ou composés, permettant de prévenir la survenue d'une lipointoxication au sein des membranes biologiques, typiquement l'accumulation cellulaire d'acides gras, en particulier d'acides gras saturés, ou de traiter une lipointoxication installée en agissant sur le phénomène communément altéré dans l'ensemble des tissus lipointoxiqués : la plasticité membranaire.

### 3. Objectifs de l'invention

La présente invention a pour objectif de pallier des inconvénients de l'art antérieur, exposés notamment ci-avant.

En particulier, l'invention a pour objectif de prévenir et/ou de traiter chez un sujet une lipointoxication par hypoxie liée à la présence en excès dans les membranes biologiques de cellules non adipocytaires, d'acides gras saturés et/ou de stérols, ladite lipointoxication par hypoxie étant associée chez ledit sujet à une pathologie pulmonaire. L'objectif de l'invention, est donc à de prévenir et/ou de traiter chez un sujet une pathologie pulmonaire, particulièrement une pathologie pulmonaire entraînant une insuffisance respiratoire, plus particulièrement une pathologie pulmonaire étant la mucoviscidose ou une broncho-pneumopathie chronique obstructive. L'invention a ainsi en particulier pour objectif de prévenir et/ou de traiter la mucoviscidose. L'invention a aussi en particulier pour objectif de prévenir et/ou de traiter les broncho-pneumopathies chroniques obstructives. L'invention permet de limiter, voire de prévenir, les dysfonctionnements ou l'apoptose des cellules non adipocytaires lipointoxiquées par hypoxie, associés notamment par la diminution ou suppression de la fluidité de leur membrane plasmique et/ou de la membrane de leurs organelles. L'objectif de l'invention, selon au moins un mode de réalisation, est de fournir un composé susceptible de prévenir et/ou de traiter chez un sujet une lipointoxication par hypoxie telle que définie plus haut, 2. tout en étant non toxique pour les cellules incapables de synthétiser des lipides neutres, typiquement des triglycérides et/ou des stérols estérifiés. L'invention a notamment pour objectif de fournir un composé susceptible de prévenir et/ou de traiter chez un sujet ladite lipointoxication par hypoxie, étant alternatif, voire supérieur à l'acide oléique dans ses propriétés, tout en étant non-toxique. L'invention a ainsi en particulier pour objectif de fournir un composé susceptible de prévenir et/ou de traiter chez un sujet cette lipointoxication par hypoxie, tout en étant non-toxique pour les cellules épithéliales bronchiques.

### 4. Résumé de l'invention

Ces objectifs, ainsi que d'autres qui apparaitront plus clairement par la suite, sont atteints à l'aide des composés, compositions et utilisations correspondants, selon la présente invention.

L'invention concerne une nouvelle classe de molécules destinées à la prévention et/ou au traitement des pathologies associées à une lipointoxication par les acides gras, typiquement les acides gras à longue chaine saturée et/ou *trans.* Par acide gras à longues chaines, on entend typiquement les acides gras dont la chaine carbonée comprend au moins 14 atomes de carbone, typiquement entre 14 et 24 atomes de carbone, par exemple au moins 16 ou au moins 18 atomes de carbone, typiquement entre 14 et 22 ou entre 14 et 18 atomes de carbone.

La lipointoxication peut se manifester par une inversion du rapport AGS/acides gras insaturés (AGI) dans les phospholipides présents au sein des membranes biologiques, les AGS devenant majoritaires, voire remplaçant complètement les AGI. Ces molécules peuvent être destinées à la prévention et/ou au traitement d'une dyslipidémie, du syndrome métabolique, d'un syndrome ou d'une anomalie caractéristique du syndrome métabolique, de préférence à la prévention et/ou au traitement du diabète de type 2.

Les molécules de l'invention peuvent être destinées à la prévention ou au traitement d'une lipointoxication d'origine endogène (ou lipointoxication « endogène »), particulièrement une lipointoxication par hypoxie (ou lipointoxication « hypoxique »). Par « hypoxie », on entend une inadéquation entre les besoins tissulaires en oxygène et les apports, entraînant un état d'oxygénation insuffisante de certaines cellules, certains tissus et/ou organes. En condition de normoxie, la proportion en O₂ et en N₂ est typiquement de 95% et 5%. Ainsi, les molécules de l'invention sont destinées à la prévention ou au traitement d'une pathologie pulmonaire, particulièrement une pathologie pulmonaire associée à une insuffisance respiratoire, plus particulièrement à une pathologie pulmonaire associée à une insuffisance respiratoire choisie parmi la mucoviscidose ou les BCPO.

En effet, il peut être distingué les lipointoxications d'origine exogène (ou lipointoxications « exogènes ») et les lipointoxications d'origine endogène (ou lipointoxications « endogènes »). Les lipointoxications exogènes sont induites par une surexposition des cellules à une source environnementale d'AGS. Les lipointoxications endogènes sont induites, quant à elles, par une dérégulation de l'équilibre endogène entre AGI et AGS. Ces lipointoxications apparaissent notamment lorsqu'une cellule n'est plus en mesure de contrôler, ou de réguler, son contenu en acides gras, de manière spatiale ou temporelle, en fonction des besoins de balance entre AGS et AGI, et des contraintes de plasticité membranaire spécifique à un compartiment endomembranaire considéré. A l'aide d'un modèle cellulaire levure cultivé dans des conditions mimant l'hypoxie, les inventeurs ont démontré que les levures accumulent des AGS (par exemple, par non conversion des AGS endogènes en AGI) et que la lipointoxication induite provoque un certain nombre d'effets délétères, tels qu'un stress du réticulum endoplasmique, un déclenchement de la réponse UPR (unfolded protein response), un défaut de vésiculation et un défaut de trafic vésiculaire dans les phases distales de la voie de sécrétion des protéines.

Les inventeurs sont partis du constat que les tableaux cliniques des patients atteints de mucoviscidose ou de BPCO partageaient un certain nombre de similitudes. Parmi ces points communs figurent (1) une exacerbation de l'inflammation dans les tissus pulmonaires, (2) un encombrement des voies respiratoires lié à l'altération des propriétés de rhéologie du mucus, (3) une forte prédisposition au déclenchement de l'apoptose et (4) une hypertension des bronches. Ils ont associé à ces différents points la présence d'une lipointoxication aux AGS et, au regard de la littérature, ont proposé que cette caractéristique puisse constituer l'élément clé duquel découlerait l'ensemble des symptômes. Afin de confirmer cette hypothèse, et afin d'identifier une possible solution thérapeutique, les inventeurs ont alors mené un certain nombre de recherches expérimentales.

Dans un premier temps, la lipointoxication de tissus bronchiques fraichement dissociés, notamment par l'acide palmitique, a été mise en évidence, par analyse du contenu en acides gras des phospholipides purifiés, à partir de biopsies bronchiques saines ou issues de patients atteints de mucoviscidose ou de BPCO. De plus, il a été proposé que cet état soit corrélé aux conditions d'insuffisance respiratoire, et à l'hypoxie, sur la base du mode d'action des désaturases intracellulaires, enzymes impliquées dans la conversion des AGS endogènes en AGI et dépendantes de l'oxygène.

Dans un second temps, ces conclusions ont été validées à l'aide d'un modèle *in vitro* reconstituant artificiellement les lipointoxications observées dans les biopsies de patients. Ce modèle a permis de valider l'implication de l'insuffisance respiratoire et, *a fortiori,* de l'hypoxie dans l'induction de formes de lipointoxications par des AGS dans les épithéliums bronchiques.

Dans un troisième temps, il a été observé que la lipointoxication des cellules épithéliales bronchiques par de l'acide palmitique déclenche la voie UPR et *in fine* l'apoptose. Les inventeurs ont également démontré que les composés et compositions selon la présente invention inhibent significativement le déclenchement de l'apoptose, et que ces composés sont conséquemment utiles pour prévenir et/ou traiter des lipointoxications, notamment une lipointoxication par hypoxie. En cela, ces composés et compositions selon l'invention peuvent en particulier être avantageusement utilisés pour prévenir et/ou traiter une pathologie choisie parmi les pathologies pulmonaires, notamment la mucoviscidose ou les broncho-pneumopathies chroniques obstructives.

De manière annexe, les inventeurs ont démontré que les composés et compositions selon la présente invention exercent un effet sur les bronches des patients atteints de la mucoviscidose ou de BPCO. En cela, les composés et compositions selon l'invention peuvent en particulier être avantageusement utilisés pour prévenir et/ou traiter l'hypertension bronchique associée aux pathologies pulmonaires, notamment la mucoviscidose ou les broncho-pneumopathies chroniques obstructives.

Un avantage considérable que présentent les molécules (ou composés) de l'invention, par rapport aux AGI, en particulier l'acide oléique, utilisés dans l'art antérieur pour compenser un excès en AGS, est que contrairement à ces derniers, elles n'engendrent aucune toxicité cellulaire, en particulier aucune toxicité sur les cellules incapables de synthétiser des lipides neutres, typiquement les cellules non-adipocytaires, par exemple les cellules pancréatiques et les cellules épithéliales bronchiques.

Les molécules de l'invention présentent un autre avantage majeur en ce que, contrairement aux AGI utilisés de manière préventive dans l'art antérieur, elles peuvent également être utilisées de manière thérapeutique en raison de leur aptitude à rétablir la fonctionnalité cellulaire, par exemple en agissant sur la plasticité membranaire. Elles peuvent ainsi avantageusement être utilisées pour traiter une lipointoxication installée, c'est-à-dire une lipointoxication responsable d'un dysfonctionnement cellulaire détectable, typiquement d'une altération de la capacité voire d'une incapacité de la cellule à exercer ses fonctions de vésiculation membranaire et de trafic vésiculaire, mécanismes fondamentaux de communication intracellulaire entre compartiments et indispensables à la vie des cellules. L'invention décrit ainsi un composé comprenant une tête polaire, comprenant au moins un résidu hydroxyle, sur laquelle est greffé un unique acide gras insaturé comprenant entre 16 et 24, par exemple entre 16 et 22 ou entre 16 et 20, atomes de carbone et ayant 1 à 6, par exemple 3, insaturation(s) en configuration *cis* pour une utilisation pour prévenir et/ou traiter, chez un sujet, une lipointoxication par hypoxie, ladite lipointoxication par hypoxie étant associée chez ledit sujet à une pathologie pulmonaire. La dyslipidémie - ou lipointoxication - affecte typiquement les membranes biologiques, y compris les membranes biologiques de cellules non adipocytaires. Dans la présente demande, le terme « dyslipidémie » dans son acception large et le terme « lipointoxication » sont utilisés de manière interchangeable. La dyslipidémie - ou lipointoxication - est généralement liée à la présence en excès dans lesdites membranes biologiques d'acides gras et/ou stérols. Les acides gras sont en particulier des acides gras à longues chaines saturées. La quantité d'AGS et/ou de stérols est en particulier jugée excessive lorsque par exemple en altérant la plasticité membranaire elle provoque un dysfonctionnement cellulaire. L'invention décrit un composé dont la tête polaire est de formule (I) : 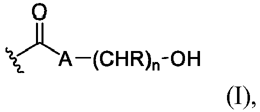 dans laquelle :
- A est un atome d'azote ou d'oxygène, de préférence un atome d'oxygène,
- n vaut 2 ou 3, de préférence n vaut 2, et
- R est n'importe quel groupement chimique,
pour une utilisation pour prévenir et/ou traiter une lipointoxication chez un sujet, typiquement une lipointoxication telle que définie ci-dessus. De tels composés peuvent être choisis parmi le 1-oléoyl-2-acétyl-sn-glycérol, 1-oléoyl-sn-glycérol-3-phosphate, 2-arachidonoylglycérol, mannide monooléate, 3-hydroxy-2,2-bis(hydroxymethyl)propyl oléate, N,N-diethanololeamide, propylène glycol monooléate, 1-oléoyl glycérol, 2-oléoyl glycérol, monoester d'acide oléique avec triglycérol, (Z)-9-Octadecenoic Acid -(2,2-Dimethyl-1,3-dioxolan-4-yl)methyl Ester, Diethylene glycol monooléate, et leurs mélanges. Les composés selon l'invention sont sélectionnés parmi le mannide monooléate, le 3-hydroxy-2,2-bis (hydromethyl)propyl oléate, le N,N-diethanololeamide, et leurs mélanges ; alternativement le N,N-diethanololeamide; alternativement le mannide monooléate, le 3-hydroxy-2,2-bis (hydromethyl)propyl oléate, et leurs mélanges .

L'invention concerne ainsi un composé tel que décrit dans le présent texte pour une utilisation pour prévenir et/ou traiter chez un sujet une pathologie pulmonaire, particulièrement une pathologie pulmonaire entraînant une insuffisance respiratoire, plus particulièrement une pathologie pulmonaire étant la mucoviscidose ou une broncho-pneumopathie chronique obstructive.

L'invention décrit par ailleurs une composition, se présentant sous la forme d'une composition pharmaceutique, d'un alicament ou d'un supplément alimentaire, comprenant au moins un composé selon l'invention. L'invention décrit également une composition pharmaceutique comprenant, outre ledit au moins un composé selon l'invention, au moins un autre composé (différent des composés selon l'invention) actif sur le plan thérapeutique (et reconnu comme tel par l'homme du métier).

### 5. Description détaillée

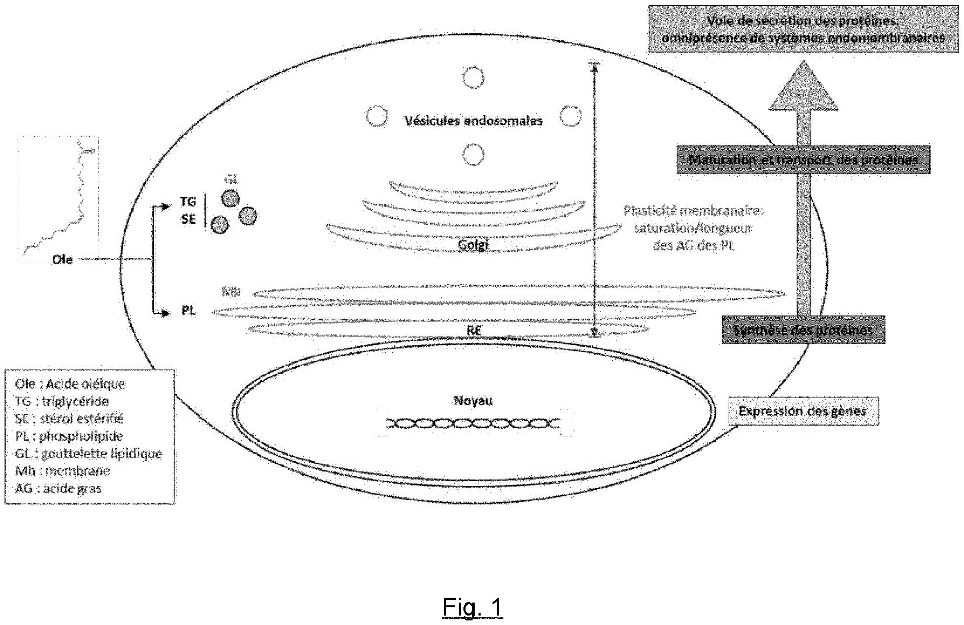

Les inventeurs ont démontré que les AGS provenant d'une source exogène (alimentation) ou endogène (hypoxie ou altération, par mutation, des étapes de désaturation des acides gras) s'accumulaient au sein des phospholipides constituant les membranes cellulaires, perturbant ainsi de nombreux processus, en altérant la fonctionnalité des organelles intracellulaires intervenant dans la voie de sécrétion des protéines (cf. Figure 1).

Pour apporter cette démonstration, les inventeurs ont développé un modèle unicellulaire simple (souche *hem1Δ* élaborée à partir de la levure de boulangerie *Saccharomyces cerevisiae*) reproduisant l'ensemble des impacts des AGS et du cholestérol observés dans les cellules de mammifères, en particulier l'ensemble des anomalies impliquées dans le développement du syndrome métabolique (Pineau *et al.,* 2008 et Pineau *et al.,* 2009).

En milieu YPG (i.e. milieu ne contenant ni Ergostérol (Erg) ni acide oléique (Ole)), la souche *hem1Δ* accumule des acides gras saturés (notamment l'acide palmitique, C16 :0) dans ses phospholipides, en particulier dans la phosphatidylcholine (PC). A noter que l'Ergostérol est le stérol majoritairement présent chez les levures et constitue donc, chez les levures, l'équivalent du cholestérol pour l'homme.

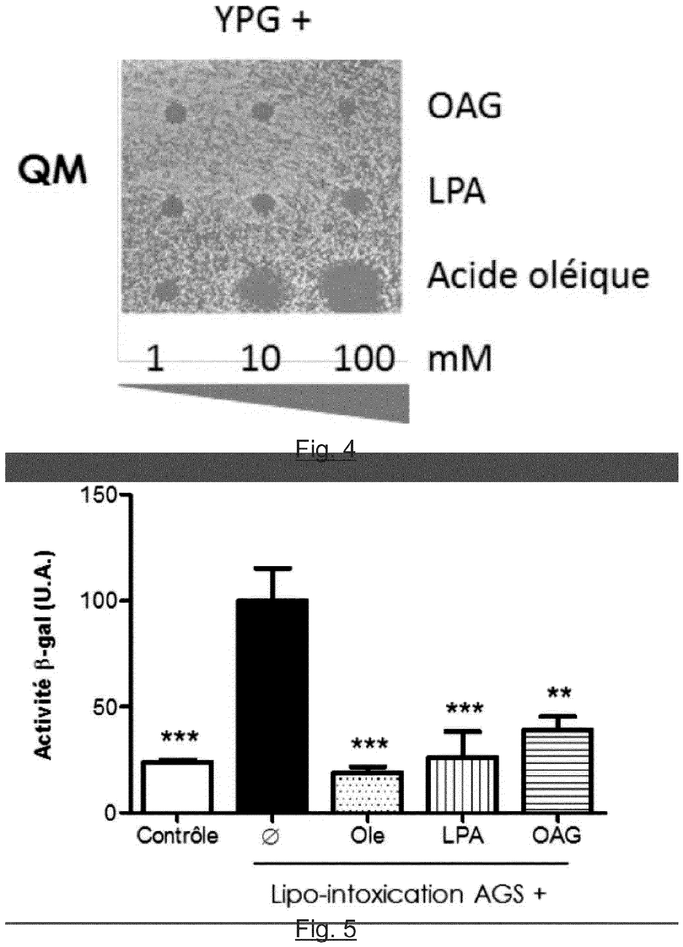

La souche QM (Petschingg *et al.,* 2009), dans laquelle les gènes codant pour les enzymes responsables de la synthèse des triglycérides et d'esters de stérols ont été délétés, est quant à elle incapable de transformer un apport exogène d'acides gras libres, type acide oléique C18 :1, en lipides neutres, de sorte que cet apport entraîne un stress délétère du fait de la perturbation de l'équilibre de la plasticité membranaire qu'il génère. L'utilisation de la souche QM a en particulier permis aux inventeurs de réaliser des tests de toxicité qui ont permis de démontrer clairement la toxicité de l'acide oléique dans de telles circonstances (cf. figure 4).

Plus précisément, les inventeurs ont observé sur les souches *hem1Δ* les effets négatifs de l'accumulation de phospholipides portant des chaînes saturées (PL saturés) dans les membranes des organelles intracellulaires, sur la formation des vésicules de sécrétion. Cette lipo-intoxication (endogène car le système cellulaire des souches *hem1Δ* ne synthétise plus que des AGS) perturbe l'environnement lipidique de la membrane du réticulum endoplasmique (RE), altère le processus de repliement (« misfolding ») des protéines et déclenche alors une réponse complexe dans ledit RE, réponse connue sous le nom de « Unfolded Protein Response » (UPR). Une saturation de ce système de sauvegarde entraine la mort cellulaire par apoptose. Parallèlement, les inventeurs ont pu observer des perturbations de la vésiculation de l'appareil de Golgi ainsi qu'une altération du trafic de protéines de référence (ex : Fur4p) entre l'appareil de Golgi et la membrane plasmique. Concrètement, les inventeurs ont observé une altération, due à la lipo-intoxication, de l'ensemble de la voie de sécrétion. En d'autres termes, la souche *hem1Δ* de levure leur a permis de confirmer à la fois les impacts des AGS sur le stress du RE mais également sur le trafic des protéines vers la membrane plasmique.

Le réticulum endoplasmique (RE) est impliqué dans plusieurs processus cellulaires fondamentaux, incluant la synthèse lipidique, la régulation de l'homéostasie calcique et la synthèse des protéines destinées aux différents organites et à la surface cellulaire (par exemple les protéines membranaires telles que les canaux ioniques et les transporteurs). Le RE est également le site où les protéines membranaires ou sécrétées sont assemblées et repliées. En conséquences, l'UPR joue un rôle essentiel dans le maintien de l'intégrité et de la fonctionnalité du RE, en permettant à cet organite de gérer l'accumulation de protéines mal-repliées (Kincaid & Cooper, 2007; Zhang & Kaufman, 2006). A noter que la toxicité des AGS est associée, dans les cellules β-pancréatiques (responsables de la synthèse d'insuline chez les mammifères) à l'induction de la réponse UPR (Cunha *et al.,* 2008; Diakogiannaki & Morgan, 2008; Laybutt *et al.,* 2007). Alkhateeb *et al.* (2007) et Kato *et al.* (2008) ont, en outre, observé que l'accumulation d'AGS altère l'adressage du récepteur à l'insuline et du transporteur du glucose Glut4 à la surface des cellules musculaires.

Schneider *et al.* (1999) ont observé que les membranes du réticulum endoplasmique (RE) et de l'appareil de Golgi sont constituées très majoritairement de phospholipides (PL) insaturés, alors que le taux de PL saturés augmente graduellement dans les compartiments les plus distaux dans la voie de sécrétion pour atteindre son maximum à la membrane plasmique. Des taux importants de PL insaturés se traduisent par une fluidité membranaire élevée, un paramètre crucial pour le recrutement de certaines protéines essentielles à la formation des vésicules. Un exemple canonique est fourni par les protéines de la famille des Arf-GAP1, l'une d'entre-elle étant Gcs1p chez la levure. Il a été montré que Gcs1p est un médiateur du transport vésiculaire à la fois entre l'appareil de Golgi et le RE, et entre le RE et la membrane plasmique (Robinson *et al.,* 2006). De façon intéressante, la délétion du gène GCS1 provoque une fragmentation de l'appareil de Golgi et une perturbation du trafic vésiculaire post-Golgien (Poon *et al.,* 2001), autant de phénomènes que les inventeurs ont eux-mêmes pu constater dans le modèle de levure *hem1Δ,* i.e. en condition d'accumulation d'AGS (cf. Payet *et al,* 2013.).

Les protéines de la famille Arf-GAP1 répondent à la courbure membranaire en s'adsorbant à la surface membranaire *via* un motif spécifique appelé ArfGAP1 Lipid Packing Sensor (ALPS; (Bigay *et al.,* 2005)). Concrètement, le motif ALPS ne reconnaît pas la courbure membranaire *per se,* c'est-à-dire une géométrie courbe, mais reconnaît un faible empilement des têtes polaires des phospholipides (« Loose Lipid Packing ») qui est une conséquence de la courbure membranaire (Bigay *et al.,* 2005). Les inventeurs sont parvenus à démontrer que les taux élevés de PL saturés en conditions de lipo-intoxication sont associés à une augmentation du Lipid Packing membranaire (Deguil *et al.,* 2011), et que cette augmentation altère le recrutement par l'appareil de Golgi du Gcs1p en provenance du cytoplasme (Payet *et al.,* 2013). Plus généralement, ils ont démontré que l'accumulation d'acides gras, en particulier d'AGS, dans les membranes biologiques provoquait la dérégulation fonctionnelle des organelles ou organites intra-cellulaires dont l'appareil de Golgi et le Réticulum Endoplasmique (RE), et en particulier une diminution du taux de vésiculation responsable d'une diminution de la translocation de certains transporteurs et récepteurs membranaires à la surface cellulaire.

Les lipointoxications cellulaires provoquées par les inventeurs résultent, *in vitro,* d'une exposition à une source exogène d'acides gras exclusivement sous forme saturée (lipointoxication « exogène ») ou, alternativement, d'une incapacité intrinsèque de la cellule à produire des formes insaturées des acides gras (lipointoxication « endogène »).

A l'aide de leur modèle de levure *hem1Δ,* les inventeurs ont montré que l'acide oléique (Ole), en étant métabolisé dans les phospholipides (PL) (cf. figure 1 - perte de PL à AGS en faveur de PL à AGI), permet de restaurer la plasticité de membranes préalablement lipo-intoxiquées par des AGS. Ils ont également démontré à l'aide de la souche de levure QM que l'effet bénéfique observé se limitait aux cellules ayant la capacité de tamponner un excès d'AGI exogènes sous forme de lipides neutres. Dans les cellules n'ayant pas cette capacité, le surplus d'acide oléique exogène entraîne, *in fine,* une prolifération anormale des membranes intracellulaires laquelle, en stressant les cellules, va déclencher leur apoptose.

Les inventeurs ont utilisé leur modèle de levure *hem1Δ* et la souche QM pour cribler des molécules d'intérêt susceptibles d'empêcher ou de limiter ce phénomène, idéalement de contrer l'effet toxique des acides gras saturés présents en excès et/ou mal métabolisés (i.e. estérifiés) et de corriger l'ensemble des phénomènes perturbés. Ils ont aussi découvert des molécules capables, en particulier, de restaurer une fonctionnalité cellulaire (en restaurant par exemple la fluidité membranaire) comparable à celle rencontrée dans des conditions non pathologiques.

L'efficacité des molécules présélectionnées par les inventeurs, i.e. leur capacité à restaurer une fonctionnalité cellulaire comparable à celle rencontrée dans des conditions non pathologiques, même dans le cas de dyslipidémies installées, a ensuite été testée et démontrée par ces mêmes inventeurs sur des cellules β-pancréatiques de mammifères, en particulier dans des cellules β-pancréatiques de rat (lignée BRIN-BD11).

Le sujet concerné par l'invention est un animal, typiquement un mammifère, par exemple un mammifère choisi parmi une souris, un rat, un porc et un être humain. Le sujet concerné est de préférence un être humain.

Dans le contexte de la présente description, la dyslipidémie - ou lipointoxication - dont la prévention et/ou le traitement est recherché affecte typiquement les membranes biologiques, en particulier les membranes biologiques de cellules non adipocytaires. Elle est généralement liée à la présence en excès dans lesdites membranes biologiques, d'acides gras, plus particulièrement d'acides gras à longues chaines saturées et/ou *trans,* et/ou de stérols. La dyslipidémie - ou lipointoxication - est typiquement responsable de l'intoxication des cellules non-adipocytaires à l'origine du dysfonctionnement ou de l'apoptose desdites cellules par diminution voire suppression de la fluidité de leur membrane plasmique et/ou de la membrane de leurs organelles. Selon l'invention, la lipointoxication est associée à la présence chez le sujet d'une pathologie pulmonaire, particulièrement d'une pathologie pulmonaire entrainant une insuffisance respiratoire, plus particulièrement une pathologie pulmonaire entrainant une insuffisance respiratoire étant la mucoviscidose ou une BPCO.

Ainsi qu'il ressort de la présente description, l'expression « présence en excès » d'acides gras, en particulier d'AGS, et/ou de stérols est synonyme de « lipointoxication » et désigne la présence, dans une cellule non adipocytaire, en particulier d'acides gras saturés et/ou *trans,* et/ou de stérols en une quantité suffisante pour perturber la voie de sécrétion décrite plus haut et ainsi altérer le fonctionnement cellulaire (typiquement la voie de sécrétion des protéines et en conséquence la fonction desdites protéines), voire, à un niveau supérieur, altérer en conséquence le fonctionnement de l'organe correspondant.

A l'échelle cellulaire, une lipointoxication se diagnostique typiquement par la mise en évidence d'une modification du contenu en acides gras des PL des membranes biologiques (au niveau des espèces phospholipidiques de phosphatidylcholine (PC) notamment) et, en particulier, par la déplétion des formes de PL à AGI au profit de PL à AGS. A l'image du mode opératoire décrit dans la partie expérimentale de la présente description, une telle signature lipidomique peut-être mise en évidence suite à l'extraction des lipides cellulaires totaux, à la purification de leurs phospholipides et à l'analyse de ces derniers par spectrométrie de masse (Deguil *et al.,* 2011).

Par ailleurs, cette lipointoxication cellulaire peut se manifester par l'induction de la réponse UPR (« Unfolded Protein Response »). Ainsi que le démontre la partie expérimentale, il est possible, *in vitro,* de détecter et de mesurer cette réponse UPR par l'analyse de l'expression d'un gène rapporteur (tel que le gène *lacZ* codant pour la β-galactosidase dont l'activité enzymatique peut être quantifiée) contenant dans sa séquence promotrice un ou des, par exemple 4, élément(s) de réponse à l'UPR (« UPRE ») spécifique(s) d'un gène caractéristiquement induit lors du déclenchement de ladite réponse, par exemple d'un gène choisi parmi CHOP, BiP, GRP78 et ATF4 (Laybutt *et al.,* 2007). Alternativement, le déclenchement de l'UPR en réponse à une lipo-intoxication peut être détecté et mesuré en quantifiant la proportion de formes actives de certaines protéines clés dans cette cascade d'évènements cellulaires. C'est le cas de la protéine eIF2α dont l'abondance de la forme active phosphorylée est proportionnelle à l'état d'activation de l'UPR. Comme expliqué dans la partie expérimentale, la quantité de la forme active phosphorylée peut être évaluée par densitométrie des images obtenues après western blot (Dhayal and Morgan, 2011).

Dans le contexte de la présente invention, la réponse UPR peut être avantageusement détectée ou mesurée par détection ou mesure de l'expression d'un gène ou de l'activité d'une protéine impliqué(e) dans la réponse « UPR », comme expliqué ci-dessus.

Des exemples de composés utilisables pour prévenir et/ou traiter une lipointoxication sont identifiés ci-dessous : 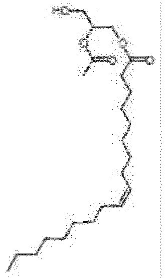 1-oléoyl-2-acétyl-sn-glycérl (**OAG**), 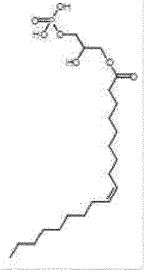 1-oléoyl-sn-glycérol-3-phosphate (acide 1-oléoyle lysophosphatidique ou **LPA**), 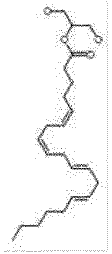 2-arachidonoylglycérol (2-AG), 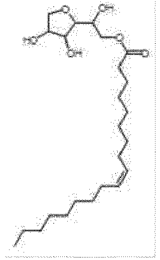 mannide monooléate, 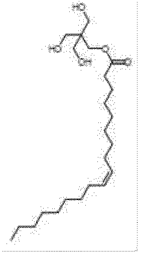 3-hydroxy-2,2-bis(hydroxymethyl)propyl oléate, 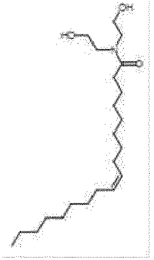 N,N-diethanololeamide, 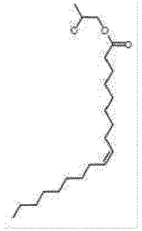 propylène glycol monooléate, 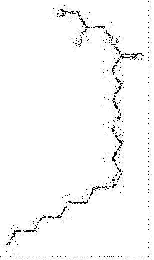 1-oléoyl glycérol, 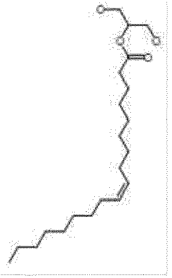 2-oléoyl glycérol, 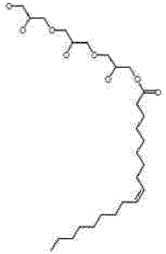 monoester d'acide oléique avec triglycérol, 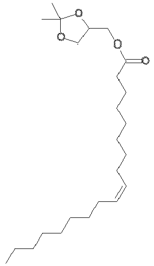 (Z)-9-Octadecenoic Acid-(2,2-Dimethyl-1,3-dioxolan-4-yl)methyl Ester 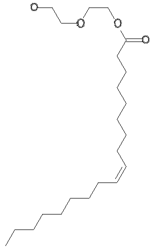 Diethylene glycol monooléate Les composés selon l'invention sont choisis parmi le mannide monooléate, le 3-hydroxy-2,2-bis(hydroxymethyl)propyl oléate et le N,N-diethanololeamide ; alternativement le N,N-diethanololeamide ; alternativement le mannide monooléate, le 3-hydroxy-2,2-bis (hydromethyl)propyl oléate, et leurs mélanges.

Un composé d'intérêt particulièrement préféré pour prévenir et/ou traiter une lipointoxication est le mannide monooléate.

Les composés d'intérêt sont utilisés dans le contexte de l'invention pour prévenir et/ou traiter les lipointoxications telles que la lipointoxication hypoxique, typiquement en restaurant la fluidité des membranes biologiques. Une caractéristique avantageuse de ces composés est que, à la différence des AGI utilisés dans l'art antérieur, ils sont non toxiques pour les cellules incapables de synthétiser des lipides neutres, typiquement des triglycérides et/ou des stérols estérifiés. Ces composés sont en particulier non toxiques pour les cellules pancréatiques (cellules β-pancréatiques et cellules α-pancréatiques). Ils sont également de préférence non toxiques pour les cellules rénales, hépatiques, cardiaques et musculaires. Ils sont également de préférence non toxiques pour les cellules épithéliales bronchiques. Ils sont en outre de préférence avantageusement capables de restaurer la fonctionnalité d'une cellule lipointoxiquée ainsi que, le cas échéant, celle des organes impliqués, tels que les voies respiratoires, et notamment les bronches.

Un composé d'intérêt typique de l'invention présente avantageusement les propriétés suivantes :
- il restaure la croissance d'un mutant *hem1Δ* de levure *Saccharomyces cerevisiae* lipointoxiqué,
- il réduit ou supprime la réponse UPR (« Unfolded Protein Response »),
- il n'est pas toxique pour un mutant QM de levure *Saccharomyces cerevisiae,* et/ou
- il réduit ou supprime la mort cellulaire par apoptose d'une cellule de mammifère lipointoxiquée .

Des composés particuliers utilisés dans le contexte de l'invention sont capables de restaurer la croissance d'un mutant *hem1Δ* de levure *Saccharomyces cerevisiae* lipointoxiqué et/ou de réduire ou supprimer la réponse UPR (« Unfolded Protein Response »), typiquement la réponse UPR induite par une lipo-intoxication (que cette dernière soit de nature endogène ou exogène).

Des composés particuliers utilisés dans le contexte de l'invention sont non toxiques pour les levures de souche QM.

Des composés particuliers utilisés dans le contexte de l'invention sont en mesure de réduire ou supprimer la mort cellulaire par apoptose de cellules de mammifère lipointoxiquées.

Dans un mode de réalisation préféré de l'invention, les composés d'intérêt sont utilisés pour prévenir et/ou traiter une pathologie pulmonaire, particulièrement d'une pathologie pulmonaire entrainant une insuffisance respiratoire, plus particulièrement une pathologie pulmonaire entrainant une insuffisance respiratoire étant la mucoviscidose ou une BPCO.

Dans un mode de réalisation préféré de l'invention, au moins un composé d'intérêt tel que décrit dans le présent texte est utilisé pour prévenir et/ou traiter la mucoviscidose ou une BPCO. Le mannide monooléate est un exemple de composé d'intérêt utilisé de manière préférée pour prévenir et/ou traiter la mucoviscidose ou une BPCO.

Ce au moins un composé d'intérêt peut être utilisé, dans un mode de réalisation particulier de l'invention, en combinaison avec un composé distinct connu de l'homme du métier et utilisé classiquement dans la prévention et/ou le traitement de la mucoviscidose ou une BPCO, ledit composé distinct étant choisi de préférence parmi les composés mucolytiques, les antibiotiques, les correcteurs de la protéine CFTR. L'invention concerne par ailleurs une composition se présentant sous la forme d'une composition pharmaceutique, d'un alicament, d'un supplément ou d'un complément alimentaire, comprenant au moins un composé d'intérêt selon l'invention. L'invention concerne également une composition pharmaceutique comprenant outre ledit au moins un composé d'intérêt selon l'invention, au moins un autre composé (différent des composés d'intérêt utilisés dans le contexte de l'invention pour prévenir ou traiter une lipointoxication par hypoxie sans induire de toxicité sur les cellules non-adipocytaires) actif sur le plan thérapeutique (et reconnu comme tel par l'homme de l'art), en particulier un composé actif dans la prévention ou le traitement d'un symptôme ou d'une anomalie caractéristique d'une pathologie pulmonaire, associée notamment à une insuffisance respiratoire.

L'invention concerne également une composition telle que décrite dans le présent texte pour une utilisation pour prévenir et/ou traiter une lipointoxication par hypoxie, typiquement une pathologie choisie parmi les pathologies pulmonaires, de préférence pour prévenir et/ou traiter la mucoviscidose ou les BPCO.

Le terme « traitement » désigne le traitement curatif, symptomatique ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression ou prévenir une progression plus en avant de la maladie, améliorant ainsi la condition des sujets. Les composés de la présente invention peuvent enfin être administrés en « prévention » aux sujets non malades, mais qui pourraient développer normalement la maladie ou qui ont un risque important de développer la maladie.

Le ou les composés d'intérêt ou compositions selon l'invention peuvent être administrés de différentes manières et sous différentes formes.

Ainsi, dans un mode de réalisation typique, le ou les composés d'intérêt sont administrés au sujet, ensemble ou séparément, et le ou les composés d'intérêt ou compositions selon l'invention sont administrés de manière continue ou séquentielle, une ou plusieurs fois par jour (administration quotidienne), une ou plusieurs fois par semaine (administration hebdomadaire), ou une ou plusieurs fois par mois (administration mensuelle), pendant toute la durée du traitement, i.e. jusqu'à l'amélioration des symptômes de la pathologie traitée, de préférence la disparition de tout ou partie desdits symptômes.

Si nécessaire, la dose journalière peut par exemple être administrée en deux, trois, quatre, cinq, six ou plus, prises par jour ou par sous-doses multiples administrées par intervalles appropriés pendant la journée.

Ces composés ou compositions peuvent être par exemple administrés de manière systémique, par voie orale, parentérale, par inhalation ou par injection, comme par exemple par voie intraveineuse, intra-péritonéale, intra-musculaire, sous-cutanée, trans-dermique, intra-artérielle, etc. S'agissant d'un traitement à long terme, la voie d'administration préférée sera sublinguale, orale, intra-péritonéale, ou transcutanée.

Les compositions peuvent être formulées sous forme de suspensions injectables, huiles, suppositoires, gélules, capsules, aérosols, etc., éventuellement au moyen de formes galéniques ou de dispositifs assurant une libération prolongée et/ou retardée. Pour les injections, les composés sont généralement conditionnés sous forme de suspensions liquides, qui peuvent être injectées au moyen de seringues ou de perfusions, par exemple.

Il est entendu que le débit et/ou la dose injectée peuvent être adaptés par l'homme du métier en fonction du patient, de la pathologie, du mode d'administration, etc. En général, la dose journalière du composé sera la dose minimum pour obtenir l'effet thérapeutique.

La quantité de composé présente dans la composition thérapeutique peut être modulée de façon à obtenir un taux circulant de principe actif nécessaire à l'obtention de l'effet thérapeutique désiré pour un patient particulier, une composition, un mode d'administration, et ce de préférence sans toxicité pour le patient. La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

Typiquement, les composés sont administrés à des doses pouvant varier entre 1 µg et 2 g par administration, préférentiellement de 0,1 mg à 1 g par administration. D'autre part, les compositions selon l'invention peuvent comprendre, en outre, d'autres agents ou principes actifs comme expliqué précédemment. Les compositions selon l'invention peuvent aussi comprendre un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc.

Les figures et exemples suivant illustrent l'invention sans en limiter la portée.

### 6. Exposé des figures

Les figures suivantes sont décrites :
**Figure 1** **: Voie de sécrétion et plasticité membranaire**
   Suite à leur synthèse, les protéines membranaires ou sécrétées (« outils » moléculaires des cellules) doivent subir des étapes de maturation à l'intérieur des cellules. Chacune des étapes de ce processus nommé « voie de sécrétion » a lieu dans un compartiment subcellulaire spécifique (réticulum endoplasmique (RE) et appareil de Golgi notamment). L'obtention de protéines matures nécessite donc un transport intracellulaire fonctionnel entre les différents systèmes endomembranaires. Ce flux est influencé, entre autres, par la plasticité des membranes des compartiments intracellulaires qui est, elle-même, directement corrélée à la nature des phospholipides (PL) qui composent les membranes. En particulier, il est admis que la présence d'AGS dans les PL diminue la fluidité membranaire alors que des PL arborant des AGI forment des membranes plus fluides.
   L'effet bénéfique de l'acide oléique (Ole) est observé sur les cellules ayant la capacité de tamponner un excès d'AGI exogènes sous forme de lipides neutres (triglycérides (TG) ou stérols estérifiés (SE) stockés sous forme de gouttelettes lipidiques (GL)). Dans les cellules n'ayant pas cette capacité, le surplus d'acide oléique exogène entraîne *in fine* une prolifération des membranes intracellulaires, laquelle en provoquant un stress cellulaire va déclencher l'apoptose.

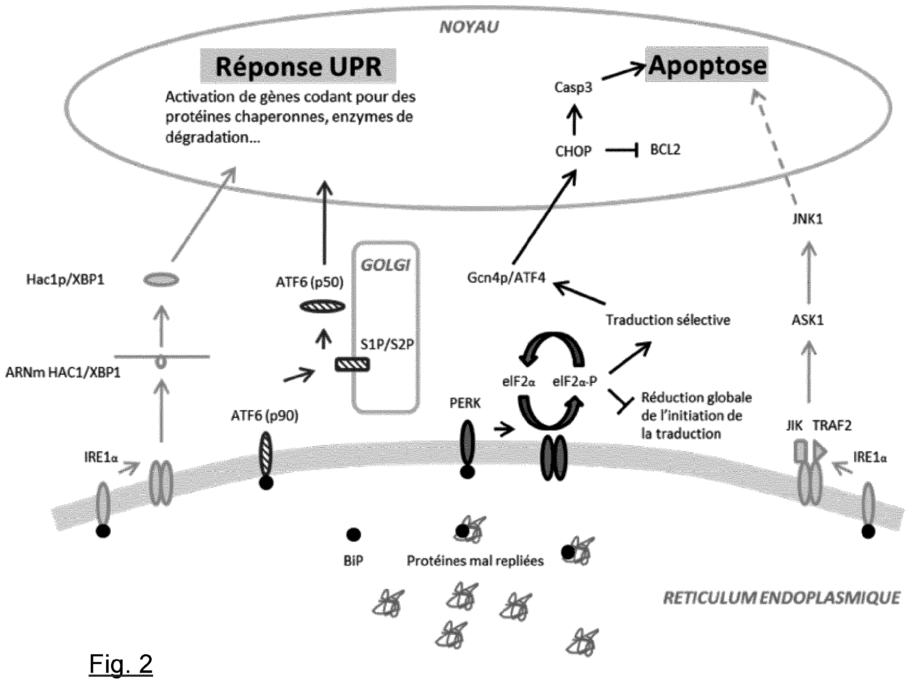

**Figure 2** **: Les voies de l'UPR chez les eucaryotes supérieurs** (Pineau & Ferreira, 2010)

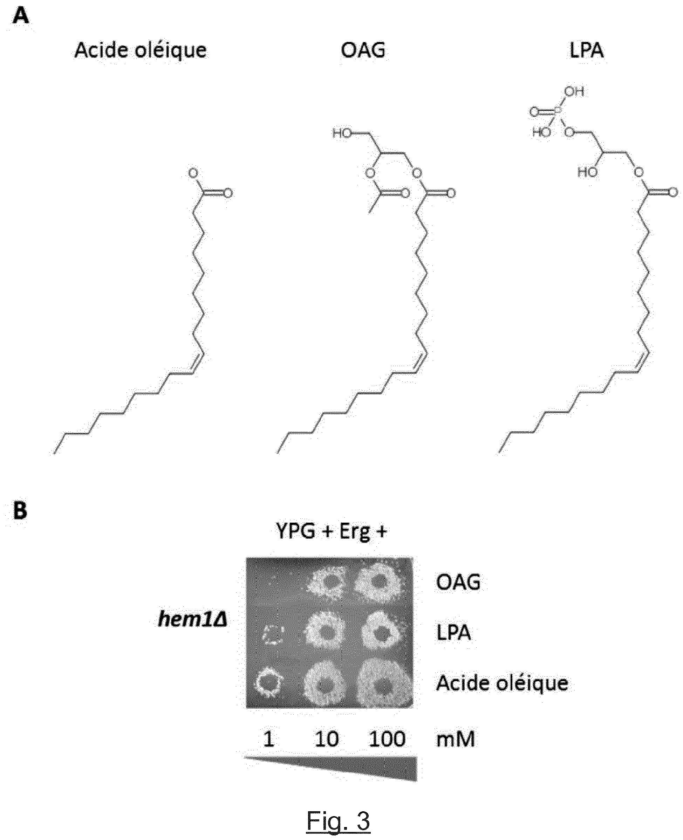

**Figure 3** **: L'acide oléique, l'OAG et le LPA restaurent la croissance de levures lipointoxiquées**
Figure 3A - Structures de l'acide oléique de l'OAG et du LPA.
Figure 3B - Les levures *hem1Δ* ont été cultivées dans des conditions d'accumulation d'AGS, comme indiqué. Des gouttes de 5µl d'OAG, de LPA ou d'acide oléique, aux concentrations indiquées, ont ensuite été déposées à la surface du milieu gélosé. La restauration de croissance des levures *hem1Δ* est constatée par la formation de colonies, après 3 jours.
**Figures 4** **: l'OAG et le LPA ne sont pas toxiques pour des cellules ne synthétisant pas de triglycérides**
   Des gouttes de 5µl d'OAG, de LPA ou d'acide oléique ont été déposées, à partir de solutions stock aux concentrations indiquées, à la surface d'un milieu gélosé sur lequel avait été préalablement étalée la souche QM. Après trois jours, des halos d'inhibition de croissance (absence de colonies) peuvent être observés dans le cas de l'acide oléique. Ces halos ne sont, en revanche, pas observés en présence de LPA ou d'OAG.
**Figures 5** **: L'OAG et le LPA réduisent la réponse UPR dans des levures lipointoxiquées**
   Une construction plasmidique portant un gène de fusion, correspondant à la séquence codante du gène *LacZ* placée sous dépendance d'un promoteur artificiel contenant 4 éléments UPR (UPRE), a été introduite dans une souche *hem1Δ* de levures, comme décrit dans Pineau *et al.* (2009). Lors d'une induction de la réponse UPR, le facteur de transcription Hac1p/XBP1p est activé et se fixe sur les éléments UPR du gène de fusion, entraînant la transcription du gène *LacZ. LacZ* codant pour la β-galactosidase, le niveau d'induction de l'UPR est donc mesuré par détection de l'activité enzymatique correspondante. La souche *hem1Δ* de levures a été cultivée dans un milieu liquide induisant l'accumulation d'AGS sans autre addition (Ø), ou dans le même milieu supplémenté de 200µM d'acide oléique, d'OAG ou de LPA, comme indiqué. U. A. : unités arbitraires.

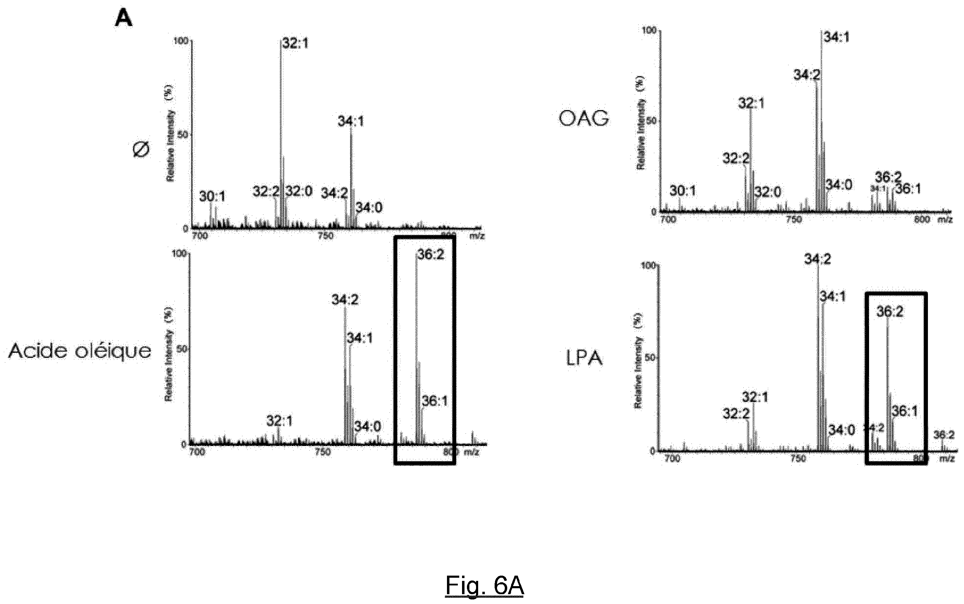

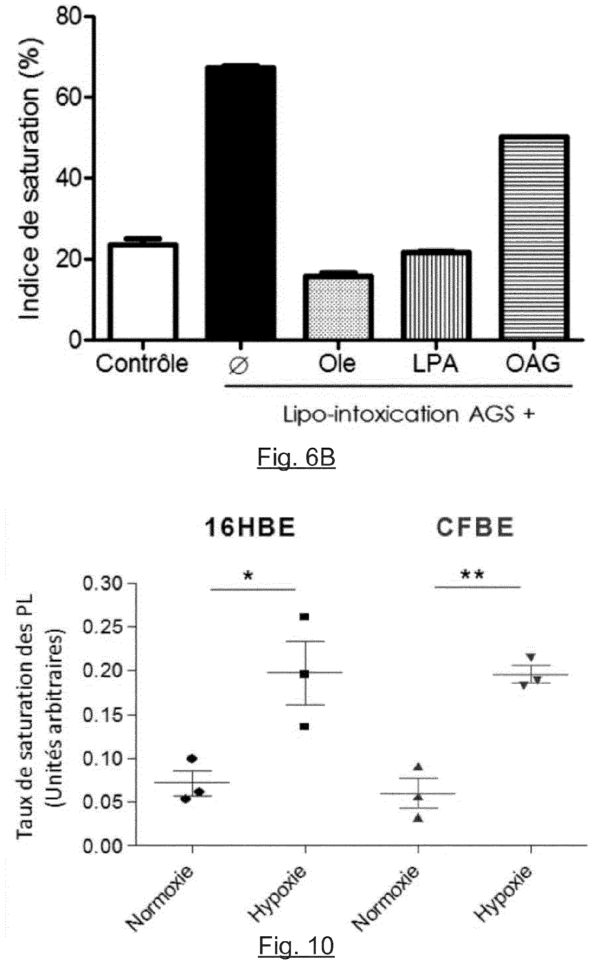

**Figures 6****: L'OAG ne restaure pas la production de phospholipides di-insaturés, contrairement à l'acide oléique et au LPA, dans des levures lipointoxiquées**
   Les levures *hem1Δ* ont été cultivées dans des conditions standard (Contrôle) ou dans des conditions d'accumulation d'AGS, sans (Ø) ou avec ajout d'acide oléique, de LPA ou d'OAG à 100 µM, comme indiqué.
Figure 6A - Après 7h d'incubation, les phospholipides (PL) ont été extraits et les différentes espèces de phosphatidylcholine (PC), qui constitue le PL majoritaire, ont été analysées par spectrométrie de masse en mode positif, selon Pineau *et al.* (2008). L'espèce 36 :2, correspondant à une PC contenant 2 chaînes d'acide oléique, est encadrée. Comme il est possible de le voir, l'addition d'acide oléique ou de LPA résulte en une augmentation du taux de cette espèce, ce qui n'est pas le cas pour l'OAG.
Figure 6B - L'ensemble des espèces de PL détectables dans ces conditions de spectrométrie de masse a été analysé pour quantifier de manière globale le contenu en formes AGS. L'indice de saturation ainsi obtenu a permis de mettre en évidence que l'OAG, contrairement à l'acide oléique et au LPA, ne restaure pas un indice de saturation comparable à celui observé dans la condition Contrôle.

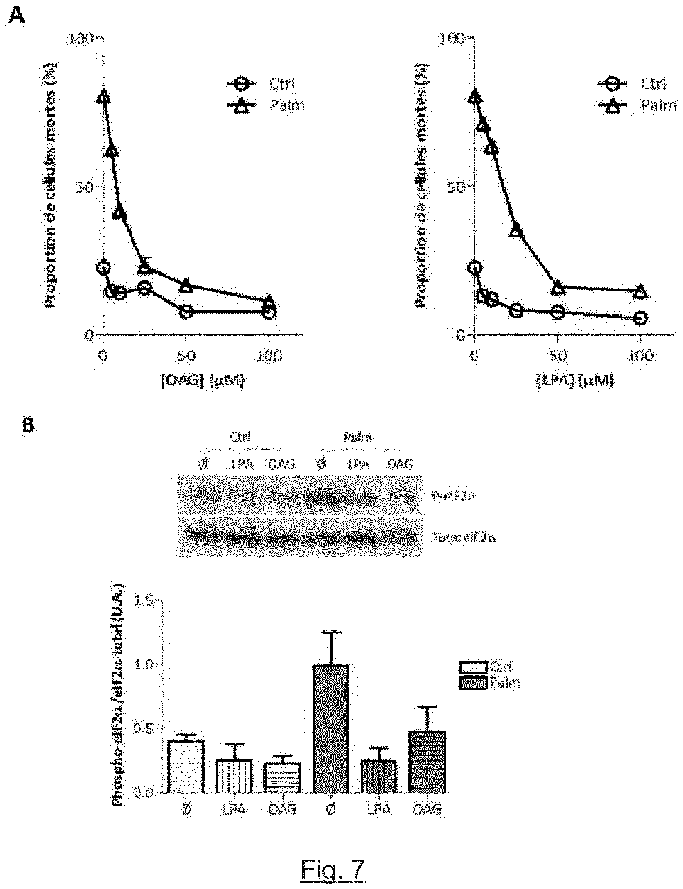

**Figures 7** **: L'OAG et le LPA préviennent l'apoptose des cellules β-pancréatiques en présence d'acides gras saturés, en réduisant le taux d'induction de l'UPR.**
   Des cellules β-pancréatiques BRIN-BD11 ont été cultivées dans des conditions contrôle ou en présence d'une source exogène d'acides gras saturé (acide palmitique, 200µM), comme décrit par Dhayal & Morgan (2011) afin de générer des conditions de lipointoxication, avec ou sans addition d'OAG ou de LPA.
Figure 7A - La proportion de cellules mortes a été estimée en absence (Ctrl) ou en présence d'acide palmitique (Palm), pour des concentrations croissantes d'OAG ou de LPA.
Figure 7B - Les taux de phosphorylation d'eIF2α ont également été analysés dans les différentes conditions par western blot, en absence (Ø) ou en présence d'OAG ou de LPA, et normalisés aux quantités d'eIF2α totales. Le taux de phosphorylation étant corrélé à l'intensité de la réponse UPR, cette expérience démontre que l'OAG réduit l'UPR induite par l'accumulation de palmitate. U. A. : unités arbitraires.

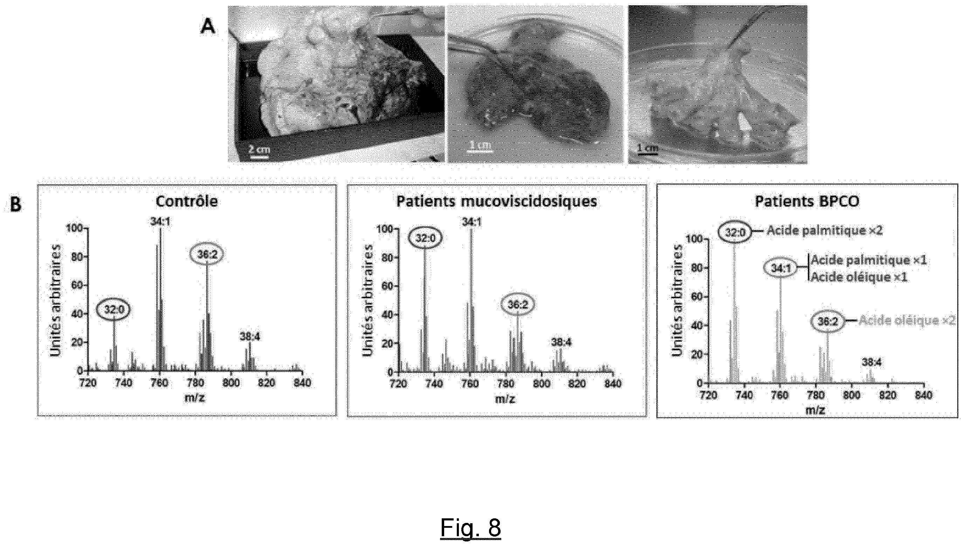

**Figures 8****: Les tissus bronchiques de patients atteints de mucoviscidose ou de BCPO sont lipointoxiqués par des AGS**
Figure 8A - Des biopsies pulmonaires ont été dissociées afin de n'en conserver que la composante bronchique (séquence de gauche à droite).
Figure 8B - Les lipides totaux des cellules épithéliales bronchiques ont été extraits, les phospholipides ont été purifiés puis les différentes espèces de phosphatidylcholine (PC), qui constituent les PL majoritaires, ont été analysées par spectrométrie de masse en mode positif, selon Pineau *et al.* (2008). Les espèces 32 :0 et 36 :2, correspondent à des PC contenant 2 chaînes d'acide palmitique et 2 chaînes d'acide oléique. Comme il est possible de le voir, le rapport AGS/AGI s'inverse lorsque des PC de biopsies contrôle sont comparées à des PC de biopsies de patients atteints de mucoviscidose ou de BPCO.

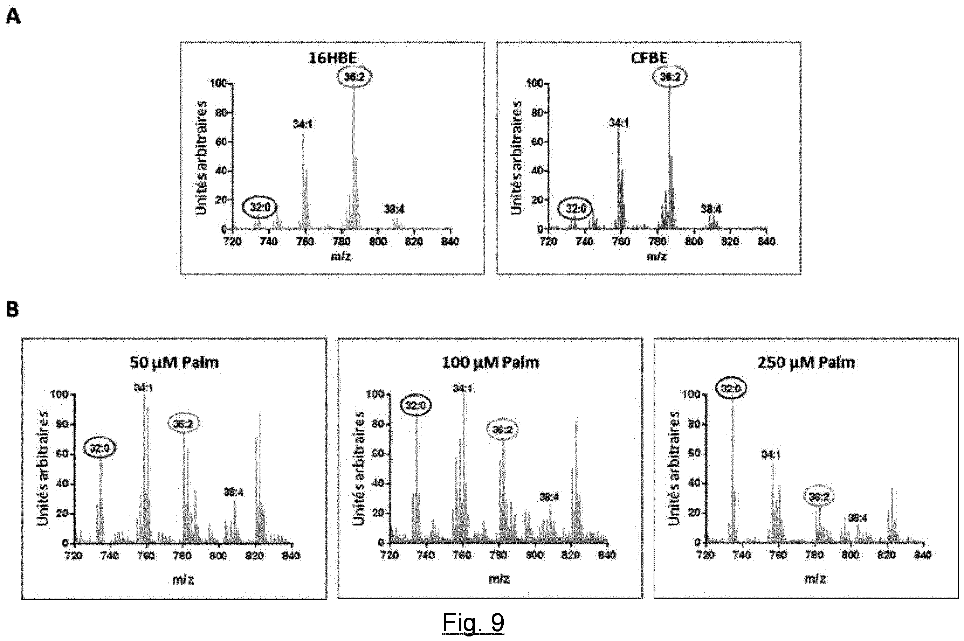

**Figures 9** **: Induction de la lipointoxication par des AGS dans des lignées cellulaires d'épithélium bronchiques, 16HBE et CFBE**
Figure 9A - Les lignées de cellules épithéliales bronchiques humaines 16HBE et CFBE ont été utilisées pour tester le contenu en acide gras de leurs phospholipides, dans des conditions de culture *in vitro.* Contrairement au profil lipidomique observé pour des biopsies pulmonaires de patients muvoviscidosiques ou atteints de BPCO (voir Figure 7B), le rapport AGS/AGI dans les PC des lignées 16HBE et CFBE indique une absence de lipointoxication.
Figure 9B - Des CFBE ont été cultivées pendant 16h avec des quantités croissantes d'acide palmitique. L'analyse du profil lipidomique des cellules dans de telles conditions révèle que des apports exogènes en AGS miment les lipointoxications observées chez des patients atteints de mucoviscidose (100 µM Palm) ou de BPCO (250 µM Palm).
**Figures 10** **: Des conditions d'hypoxie induisent, *in vitro,* la lipointoxication des lignées cellulaires 16HBE et CFBE, reconstituant artificiellement les lipointoxications hypoxiques observées dans les biopsies de patients**
   Les 16HBE et les CFBE ont été cultivées pendant 24h dans des conditions standard (normoxie : 95 % O₂ + 5 % CO₂) ou dans un environnement anoxique (hypoxie : 95 % N₂ + 5 % CO₂). Après avoir extrait les lipides totaux et purifié les phospholipides, le contenu global en acides gras a été analysé. Les rapports AGS/AGI ont été calculés afin de déterminer les taux de saturation des phospholipides dans chacune des conditions. Les résultats indiquent que la lipointoxication peut être induite, *in vitro,* par une hypoxie artificielle.

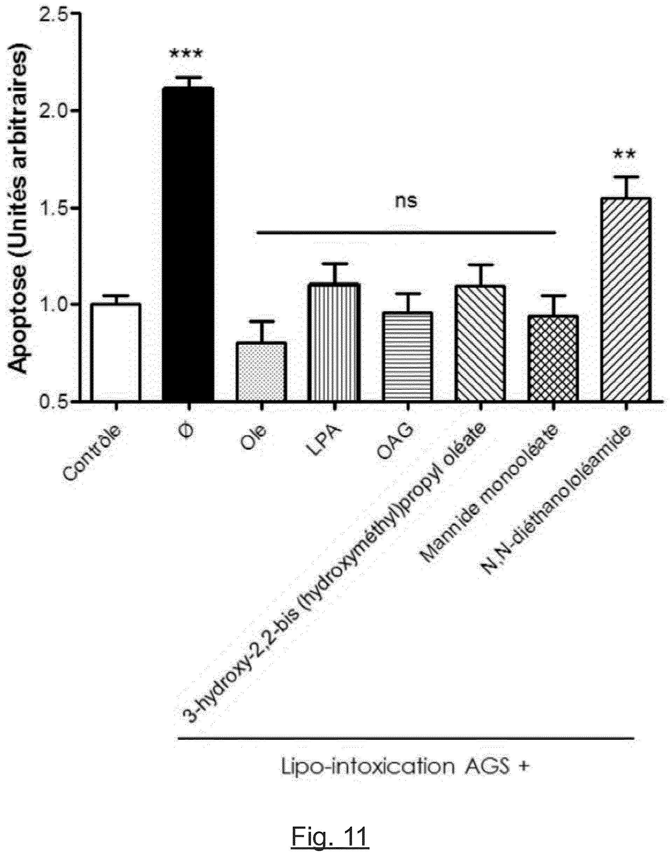

**Figure 11** **: Les composés anti-AGS contre l'influence pro-apoptotique de la lipointoxication dans les cellules épithéliales bronchiques**
   Les CFBE ont été cultivées dans des conditions standard (Contrôle) ou ont été soumises à une source d'AGS exogènes (acide palmitique 250 µM), pendant 16h, sans (Ø) ou avec l'ajout de 100 µM des composés d'intérêt, comme indiqué. Les cellules ont alors été lysées et l'apoptose a été quantifiée, comme indiqué dans la partie Exemples.

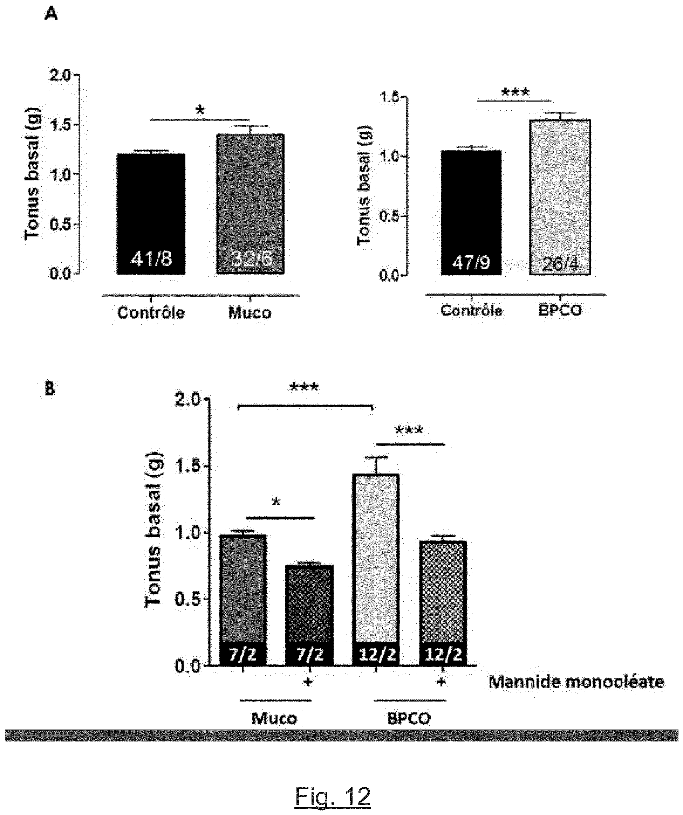

**Figures 12** **: Le mannide monooléate dissipe l'hypertension bronchique des tissus pathologiques**
Figure 12A - Des anneaux bronchiques de patients sains (Contrôle), atteints de mucoviscidose (muco) ou de broncho-pneumopathie chronique obstructive (BPCO) ont été disséqués puis leur tonus de base a été mesuré. Les résultats indiquent que les deux pathologies respiratoires sont corrélées à une hypertension des bronches.
Figure 12B - La même opération a été réalisée suite à une pré-incubation des anneaux pendant 4h à 37°C dans une solution physiologique supplémentée (+) ou non de 100 µM de mannide monooléate, comme indiqué.

Pour chaque histogramme un rapport N/n est affiché. N correspond au nombre d'anneaux testés et n au nombre de patients analysés.

### 7. Exemples

L'invention sera mieux comprise à la lecture des exemples suivants :

### A/ Souches de levures, lignées de cellules de mammifères et échantillons biologiques

Les souches de levures *Saccharomyces cerevisiae* listées dans le tableau 1 sont utilisées pour les différents tests de restauration de croissance, pour la mise en évidence des toxicités, pour l'analyse du contenu en acides gras des phospholipides cellulaires ainsi que pour les tests de déclenchement de l'« unfolded protein response » (UPR).

L'état d'activation de l'UPR et l'induction de la mort cellulaire par lipo-intoxication ont également été analysés dans des lignées β pancréatiques de rat, BRIN-BD11.

Par ailleurs, l'induction de l'apoptose, la sécrétion d'IL-8 et le profil lipidomique, en réponse à la lipointoxication aux AGS, ont également été analysés sur des lignées de cellules épithéliales bronchiques humaines 16HBE (homozygote sauvage pour le gène *CFTR*) et/ou CFBE (homozygote F508del-*CFTR*).

De plus, des expériences *ex vivo,* sur des biopsies de patients sains, atteints de mucoviscidose ou de BPCO ont été réalisées afin de déterminer les profils lipidomiques correspondant, ainsi que l'influence des composés d'intérêt sur le phénomène d'hypertension pulmonaire. Les utilisations des biopsies respectent une charte éthique définie par le Comité de Protection des Personnes (CPP) Ouest III.

**Tableau 1 : souches de levures utilisées**

| **Souche** | **Génotype** | **Origine** |
|---|---|---|
| *hem1Δ* | ***MATα** trp1 his3 ura3 leu2 hem1::LEU2* | FY1679α x FYHO4 |
| **QM** (H1246 W303) | ***MATα** are1::HIS3 are2::LEU2 dgal::KanMX4 lro1::TRP1 ADE2 ura3* | ScanBi Ltd., Alnarp, Sweden |
| **WT** (G175 W303) | ***MATa** ADE2 MET his3 leu2 ura3 trp1* | ScanBi Ltd., Alnarp, Sweden |

### B/ Lipointoxications des levures hem1Δ

La souche portant la mutation *hem1Δ* est cultivée, en conditions aérobies, sous agitation et à 28°C, dans un milieu liquide YPG^{A} (YPG (extrait de levure 1 % (m/v), peptone 1 % (m/v) et glucose 2 % (m/v)) supplémenté avec de l'acide δ-aminolevulinique (ALA) à 80 µg/mL). La lipo-intoxication par des acides gras saturés (AGS) est provoquée par déplétion en acides gras insaturés (AGI) - dont la synthèse est dépendante de la présence d'hème (groupement prosthétique de l'enzyme Ole1p notamment) - par transfert en milieu YPG⁺ (YPG supplémenté d'ergostérol à 80 µg/mL pour compenser la déplétion en stérol obtenue dans cette condition). La lipo-intoxication peut être induite sur milieu solide YPG⁺ + agar 2 % (m/v) en transférant 3500 cellules (*hem1*Δ issues d'une préculture en YPG^{A})/cm² ou, alternativement, en milieu liquide en inoculant 2.10⁶ cellules/mL de YPG⁺. Classiquement, les effets de la lipo-intoxication aux AGS sont analysés 7h après le transfert en milieu YPG⁺. La capacité d'un composé à contrer les effets délétères d'une lipo-intoxication aux AGS est, quant à elle, évaluée successivement à l'ajout de ce composé sur (ou dans) le milieu de transfert YPG⁺, après l'ensemencement avec les cellules.

### C/ Lipointoxications des cellules β-pancréatiques de rat par de l'acide palmitique

### 1) Préparation des réactifs lipidiques:

Les espèces lipidiques sont préparées dans de l'éthanol avant d'être complexées à de l'albumine de sérum bovin (BSA, initialement dépourvue d'acides gras) par une incubation de 1 heure à 37°C. Le stock de Palmitate est obtenu par l'ajout d'un volume d'éthanol avant que l'ensemble ne soit chauffé à 70°C pour homogénéisation. Les solutions d'OAG et de LPA sont préparées dans de l'éthanol 100% à température ambiante. Pour les incubations de cellules de mammifères, les concentrations finales de BSA et d'éthanol dans le milieu de culture sont respectivement maintenues à 1 et 0,5 % (m/v).

### 2) Tests de viabilité cellulaire:

La lignée de cellules β-pancréatiques (BRIN-BD11) de rat est cultivée dans du milieu RPMI-1640 complet, contenant du glucose à 11 mM et supplémenté a 10 % (v/v) de sérum de veau foetal (SVF), 2 mM de L-glutamine, 100 U/mL de pénicilline et 100 µg/mL de streptomycine. Pour chaque expérience, les cellules sont initialement ensemencées à une densité de 0,5×10⁵ cellules/mL dans des boites 6 puits pendant 24 heures. Par la suite, le milieu complet est remplacé par un équivalent dépourvu de SVF mais contenant les réactifs lipidiques d'intérêt, aux concentrations désirées, complexés à de la BSA. Dans le cas des conditions contrôles, des quantités identiques de BSA et d'éthanol sont alors utilisées. Au terme des incubations, l'ensemble des cellules (mortes et vivantes) est collecté et centrifugé à 300 g pendant 5 min. Le culot cellulaire est ensuite remis en suspension dans 200 µL de milieu puis l'ADN des cellules mortes (ayant perdu l'intégrité de leur membrane plasmique) est marqué à l'iodure de propidium (IP) en ajoutant 200 µL d'une solution d'IP à 20 µg/mL dans du tampon FACS (phosphate-buffered saline (PBS), 2 % (v/v) SVF, azoture de sodium 10 mM). Après une incubation de 10 min sur glace, les échantillons ainsi obtenus sont analysés par cytométrie en flux. Un Beckman Coulter EPICS XL MCL est utilisé pour la quantification, un canal FL3 sert à la détection des émissions de l'IP intercalé dans l'ADN et l'analyse est réalisée à l'aide du logiciel EXPO32 ADC (Applied Cytometry Systems, V 1.1 build 207).

### 3) Western blotting:

Les cellules BRIN-BD11 sont ensemencées à une densité de 0,5×10⁵ cellules/mL dans des flasques T25 pendant 24 heures. Comme indiqué précédemment, le milieu complet est alors remplacé par un équivalent dépourvu de SVF mais contenant les réactifs lipidiques d'intérêt. Après 6 heures d'incubation, l'extraction des protéines totales est réalisée à l'aide d'un tampon de lyse (Tris 20 mM, NaCl 150 mM, EDTA 1 mM et Triton-X 1 % (v/v)) contenant des inhibiteurs de protéases et de phosphatases. Ces protéines sont alors soumises à une électrophorèse en gel d'acrylamide 12 % NuPAGE® Novex® Bis-Tris Gels (Invitrogen) avant d'être transférées sur membrane de PVDF puis sondées à l'aide d'anticorps anti-phosphoeIF2α (Cell Signalling (New England Biolabs)) dilués au 1/1000^{ième}. Dans un second temps, les membranes sont décapées avec le tampon Re-Blot Plus-Strong (Millipore) avant d'être sondées une seconde fois avec des anticorps anti-eIF2α total (Cell Signalling (New England Biolabs) dilués au 1/1000^{ième}. L'analyse par densitométrie de l'abondance relative des formes phosphorylées ou non phosphorylées de la protéine eIF2α est effectuée avec le système Fluor-S Multi-imager analysis system combiné au logiciel Quantify One (Biorad UK Ltd).

### D/ Restauration de croissance

**1) Criblage de composés :** Suite à l'induction d'une lipo-intoxication aux AGS (pour des *hem1*Δ cultivées sur un milieu solide) des gouttes de 5 µL de solutions de différents composés à 10 mM dans du diméthylsulfoxide (DMSO) ou dans de l'éthanol (EtOH) sont déposées à la surface de l'agar. La capacité d'un composé à contrer l'arrêt de croissance cellulaire lipo-induit est estimée par l'apparition d'un halo de colonies de *hem1*Δ à l'emplacement du dépôt dudit composé après 3 jours de culture à 28°C (cf. Deguil *et al.,* 2011).
**2) Cinétique de prolifération :** Conjointement à l'induction d'une lipo-intoxication aux AGS (pour des *hem1*Δ en milieu liquide) différents composés sont ajoutés aux cultures à une concentration initiale de 200 µM. Le suivi de prolifération est réalisé en mesurant la densité cellulaire par spectrométrie, à intervalles de temps réguliers (toutes les heures sur la durée de l'observation). A une longueur d'onde de 600 nm, une unité de densité optique (DO_{600 nm}) correspond à 2.10⁷ cellules/mL.

### E/ Test de toxicité

Parallèlement, les souches sauvages (WT) et QM sont cultivées, en condition aérobie, sous agitation et à 28°C, dans un milieu liquide YPG avant d'ensemencer 3500 cellules par cm² de YPG + agar 2 % (m/v). Suite à ce transfert sur milieu solide, des gouttes de 1 µL de solutions de différents composés à 1, 10 et 100 mM dans du DMSO ou de l'EtOH sont déposées à la surface de l'agar. Séparément, des dépôts de DMSO et d'EtOH sont également réalisés afin d'évaluer la toxicité intrinsèque de ces deux solvants. Après 3 jours de culture à 28°C, la toxicité des composés testés est évaluée en comparant les diamètres des halos d'inhibition de croissance obtenus pour les dépôts de solvants bruts à ceux des dépôts des différentes concentrations de composés testés. Contrairement à la souche WT, la souche QM est incapable de tamponner un excès d'acide oléique exogène sous forme de lipides neutres (triglycérides (TG) ou esters de stérols (ES)) dans des gouttelettes lipidiques. Ainsi, dans le cas d'une absence de toxicité vis-à-vis de la souche WT, l'observation d'une toxicité d'un composé vis-à-vis de la souche QM indique que ce composé est perçu comme une source d'acide gras libre par les levures.

### F/ Extraction de lipides totaux

La souche *hem1Δ* est cultivée en milieu liquide YPG^{A}, YPG⁺ ou YPG⁺ + 200 µM de composé à tester, en aérobie, sous agitation et à 28°C pendant 7 h, à partir d'une concentration cellulaire initiale de 2.10⁶ cellules/mL. A l'issue de la culture, 10⁸ cellules sont collectées afin de réaliser l'extraction des lipides totaux. Après avoir mis les cellules en suspension dans 1 mL d'eau distillée à 4°C, 500 µL de billes de verre (Ø 0,6 mm) sont ajoutés et l'ensemble subit alors 3 séquences de 20 sec à 5000 tours/min dans un agitateur (les tubes sont maintenus sur glace entre chacune des 3 séquences). Le lysat cellulaire alors obtenu, complété de l'eau de rinçage des billes (1 mL), est ensuite transféré dans un tube en verre de 40 mL (Corex™) avant de réaliser l'extraction des lipides en utilisant un ratio méthanol:chloroforme 2:1 (v/v). Initialement, 6 mL de méthanol sont ajoutés et l'ensemble est vortexé pendant 30 sec puis incubé pendant 15 min à 65°C. Une fois le mélange refroidi à température ambiante, 3 mL de chloroforme sont ajoutés puis l'ensemble est à nouveau vortexé pendant 30 sec avant de laisser l'extraction se dérouler pendant 16 h. Ultérieurement, l'échantillon est centrifugé pendant 12 min à 10000 g avant de transférer le surnageant dans un nouveau tube Corex™. Après l'ajout de 2 mL de chloroforme puis de 4 mL d'eau distillée, l'ensemble est vortexé pendant 30 sec puis centrifugé pendant 8 min à 3000 g. Après élimination de la phase supérieure résultante, la phase organique inférieure est collectée dans un tube à hémolyse en verre. Finalement, le solvant est évaporé sous flux d'azote à 80°C pour obtenir les échantillons de lipides cellulaires totaux.

Dans le cas des biopsies de patients, après dissection des lobes pulmonaires et extraction des bronches, les cellules épithéliales bronchiques ont été rincées trois fois dans du PBS avant de procéder à la préparation des lipides totaux comme indiqué précédemment, à partir de 10⁵ cellules.

### G/ Purification de phospholipides et analyse par spectrométrie de masse

Les échantillons de lipides cellulaires totaux sont remis en suspension dans 1 mL de dichlorométhane en étant vortexés pendant 30 secondes. L'ensemble est déposé sur une colonne de silice (BOND ELUT-SI, 100 mg 1 mL) pré-conditionnée avec 3 mL de méthanol puis 2 mL de dichlorométhane successivement. La fraction retenue par la colonne est ensuite lavée avec 2 mL de dichlorométhane puis 3 mL d'acétone successivement. Finalement, 2 mL d'un mélange de chloroforme/méthanol/eau 50:45:5 (v/v/v) sont déposés sur la colonne et les phospholipides ainsi élués sont collectés dans un tube à hémolyse en verre. Le solvant est évaporé sous azote à 80°C pour obtenir les échantillons de phospholipides cellulaires.

Une fois remis en suspension dans 100 µL de mélange Mix⁻(isopropanol/acétonitrile/eau 2:1:1 (v/v/v) + triéthylamine 1 % (v/v)) ou de mélange Mix⁺ (isopropanol/acétonitrile/eau 2:1:1 (v/v/v) + acide formique 1 % (v/v)), les échantillons sont analysés par spectrométrie de masse (ElectroSpray Ionization-Mass pectrometry (ESI-MS)), en mode négatif ou positif respectivement, et les résultats obtenus servent à analyser le contenu en acide gras des différentes espèces de phospholipide.

### H/ Test de déclenchement de l'UPR

La souche *hem1*Δ transformée par le plasmide pPW344 [2µ URA3 4×UPRE-*LacZ* (Patil *et al.,* 2004)] est cultivée en milieu liquide YPG^{A}, YPG ou YPG + 200 µM de composé à tester, en condition aérobie, sous agitation et à 28°C pendant 7 h, à partir d'une concentration cellulaire initiale de 2.10⁶ cellules/mL. A l'issue de la culture, 10⁸ cellules sont collectées afin de quantifier l'activité beta-galactosidase (β-gal) résultante de l'expression du transgène *LacZ* (dans le cas d'une activation de l'UPR). Dans un premier temps, les cellules sont remises en suspension dans 1,5 mL de tampon Z (Na₂HPO₄ à 60 mM, NaH2PO₄ à 40 mM, KCl à 10 mM, MgSO₄ à 1 mM et β-mercaptoéthanol à 50 mM ; solution à pH 7) puis 1/15^{ième} de cette suspension est utilisé pour réaliser une mesure de DO_{600 nm}. Dans un second temps, la suspension est complétée avec 100 µL de sodium dodecyl sulfate (SDS) 0,1 % (v/v) et 200 µL de chloroforme puis vortexée en deux séquences de 30 sec successives. Après décantation, 400 µL (volume V) de la solution ainsi obtenue sont transférés dans un tube à hémolyse en verre puis complétés de 600 µL de tampon Z. 200 µL de substrat ortho-nitrophényl-β-galactoside (ONPG), à 4 mg/mL dans du tampon Z, sont alors ajoutés avant que l'ensemble soit homogénéisé au vortex puis incubé au bain marie à 30°C pour initier la réaction. Lorsque l'ensemble présente une légère teinte jaune, la réaction est interrompue (au temps t), à température ambiante, par ajout de 500 µL de Na₂CO₃ à 1M. Finalement, après avoir centrifugé les échantillons pendant 5 min à 800 g puis collecté les surnageants dans de nouveaux tubes à hémolyse en verre, les produits de la réaction (o-nitrophénol) ainsi que les débris cellulaires sont dosés par spectrométrie aux longueurs d'onde 420 et 550 nm respectivement. Pour chaque échantillon, l'activité β-gal (U) est calculée grâce à la formule U=(1000×[DO₄₂₀ₙₘ-(1,75×DO₅₅₀ₙₘ)])(t×V×DO₆₀₀ₙₘ), exprimée en unité arbitraire.

### I/ Lipointoxications in vitro des lignées cellulaires d'épithélium bronchiques, 16HBE et CFBE

**1) Lipointoxication par de l'acide palmitique exogène.** A la manière de ce qui a été présenté précédemment pour les BRIN-BD11, les 16HBE et les CFBE sont cultivées dans du MEM supplémenté de 5µg/mL de plasmocine et 10 % (v/v) de sérum de cheval, à 37°C avant d'être exposées à des concentrations d'acide palmitique de 50, 100 ou 250 µM pendant 16h et de tester les conséquences de la lipointoxication exogène.
**2) Lipointoxication hypoxique.** De manière standard, les 16HBE et les CFBE sont cultivées en condition de normoxie. De cette manière, les cellules sont entretenues dans une enceinte alimentée par un mélange composé de 95 % de O₂ et de 5 % de CO₂. Dans des conditions d'induction de l'hypoxie, les cellules sont soumises à un mélange gazeux anoxique composé de 95 % de N₂ et de 5 % de CO₂ pendant 48h avant de tester les conséquences de la lipointoxication dite hypoxique.
**3) Test de l'apoptose.** Dans des conditions de lipointoxication exogène, l'induction de l'apoptose a été analysée par utilisation du kit cell death détection kit ELISA^{PLUS} (ROCHE). Les CFBE sont ensemencées dans une boite 96 puits à une concentration de 10000 cellules par puit. Au terme des 16h de lipointoxication, les cellules sont lysées et l'apoptose est mesurée selon les instructions fournies, par quantification des oligonucleosomes cytoplasmiques qui sont révélateurs de la dégradation de l'ADN associée à l'apoptose.

### J/ Mesure du tonus basal bronchique

Après dissection des lobes pulmonaires, des anneaux bronchiques sont isolés et montés sur un dispositif d'analyse par la technique des organes isolés, dans lequel ils sont immergés dans un tampon physiologique KREBS. Suite à la stabilisation du tonus des anneaux, le tonus basal est mesuré. Alternativement, les anneaux sont incubés pendant 4h dans un tampon KREBS supplémentée de 100 µM de mannide monooléate

### REFERENCES

- Alkhateeb H, Chabowski A, Glatz JFC, Luiken JFP, Bonen A (2007) Two phases of palmitate-induced insulin résistance in skeletal muscle: impaired GLUT4 translocation is followed by a reduced GLUT4 intrinsic activity. American Journal of Physiology - Endocrinology And Metabolism 293: E783-E793
- Bigay J, Casella JF, Drin G, Mesmin B, Antonny B. ArfGAP1 responds to membrane curvature through the folding of a lipid packing sensor motif. EMBO J. 2005 Jul 6;24(13):2244-53.
- Boslem E, MacIntosh G, Preston AM, Bartley C, Busch AK, Fuller M, Laybutt DR, Meikle PJ, Biden TJ. A lipidomic screen of palmitate-treated MIN6 β-cells links sphingolipid metabolites with endoplasmic reticulum (ER) stress and impaired protein trafficking. Biochem J. 2011 Apr 1;435(1):267-76.
- Butler AE, Janson J, Bonner-Weir S, Ritzel R, Rizza RA, Butler PC. (2003) Î2-Cell Déficit and Increased Î2-Cell Apoptosis in Humans With Type 2 Diabetes. Vol. 52, pp. 102-110.
- Cnop M, Hannaert JC, Hoorens A, Eizirik DL, Pipeleers DG (2001) Inverse Relationship Between Cytotoxicity of Free Fatty Acids in Pancreatic Islet Cells and Cellular Triglycéride Accumulation. Diabetes 50: 1771-1777.
- Cunha DA, Hekerman P, Ladriere L, Bazarra-Castro A, Ortis F, Wakeham MC, Moore F, Rasschaert J, Cardozo AK, Bellomo E, Overbergh L, Mathieu C, Lupi R, Hai T, Herchuelz A, Marchetti P, Rutter GA, Eizirik DL, Cnop M. (2008) Initiation and execution of lipotoxic ER stress in pancreatic {beta}-cells. Vol. 121, pp. 2308-2318.
- Deguil J, Pineau L, Rowland Snyder EC, Dupont S, Beney L, Gil A, Frapper G, Ferreira T (2011) Modulation of Lipid-Induced ER Stress by Fatty Acid Shape. Traffic 12: 349-362
- Dhayal S, Morgan NG (2011) Structure-activity relationships influencing lipid-induced changes in eIF2alpha phosphorylation and cell viability in BRIN-BD11 cells. FEBS Lett 585: 2243-2248
- Diakogiannaki E, Morgan NG. (2008) Differential regulation of the ER stress response by long-chain fatty acids in the pancreatic β-cell. Vol. 036, pp. 959-962.
- Diakogiannaki E, Welters HJ, Morgan NG. (2008) Differential regulation of the endoplasmic reticulum stress response in pancreatic {beta}-cells exposed to long-chain saturated and monounsaturated fatty acids. Vol. 197, pp. 553-563.
- Egnatchik RA, Leamy AK, Jacobson DA, Shiota M, Young JD. ER calcium release promotes mitochondrial dysfunction and hepatic cell lipotoxicity in response to palmitate overload. Mol Metab. 2014 May 22; 3(5):544-53.
- Guo W, Wong S, Xie W, Lei T, Luo Z. (2007) Palmitate modulates intracellular signaling, induces endoplasmic reticulum stress, and causes apoptosis in mouse 3T3-L1 and rat primary preadipocytes. Vol. 293, pp. E576-586.
- Kato T, Shimano H, Yamamoto T, Ishikawa M, Kumadaki S, Matsuzaka T, Nakagawa Y, Yahagi N, Nakakuki M, Hasty AH, Takeuchi Y, Kobayashi K, Takahashi A, Yatoh S, Suzuki H, Sone H, Yamada N. (2008) Palmitate Impairs and Eicosapentaenoate Restores Insulin Secretion Through Regulation of SREBP-lc in Pancreatic Islets. Vol. 57, pp. 2382-2392.
- Katsoulieris E, Mabley JG, Samai M, Green IC, Chatterjee PK (2009) [alpha]-Linolenic acid protects renal cells against palmitic acid lipotoxicity via inhibition of endoplasmic reticulum stress. European Journal of Pharmacology 623: 107-112
- Kincaid MM, Cooper AA (2007) ERADicate ER Stress or Die Trying. Antioxid Redox Signal
- Kirby EF, Heard AS, Wang XR. Enhancing the Potency of F508del Correction: A Multi-Layer Combinational Approach to Drug Discovery for Cystic Fibrosis. J Pharmacol Clin Toxicol. 2013 Aug 28;1(1):1007.
- Kohlwein SD, Petschnigg J (2007) Lipid-induced cell dysfunction and cell death: lessons from yeast. Current hypertension reports 9: 455-461
- Laybutt DR, Preston AM, Akerfeldt MC, Kench JG, Busch AK, Biankin AV, Biden TJ (2007) Endoplasmic reticulum stress contributes to beta cell apoptosis in type 2 diabetes. Diabetologia 50: 752-763
- Listenberger LL, Han X, Lewis SE, Cases S, Farese RV, et al. (2003) Triglyceride accumulation protects against fatty acid-induced lipotoxicity. PNAS 100: 3077-3082
- Patil CK, Li H, Walter P. Gcn4p and novel upstream activating sequences regulate targets of the unfolded protein response. PLoS Biol. 2004 Aug;2(8):E246
- Payet LA, Pineau L, Snyder EC, Colas J, Moussa A, Vannier B, Bigay J, Clarhaut J, Becq F, Berjeaud JM, Vandebrouck C, Ferreira T. Saturated fatty acids alter the late secretory pathway by modulating membrane properties. Traffic. 2013 Sep 6
- Pedemonte N, Lukacs GL, Du K, Caci E, Zegarra-Moran O, Galietta LJ, Verkman AS. Small-molecule correctors of defective DeltaF508-CFTR cellular processing identified by high-throughput screening. J Clin Invest. Sep;115(9):2564-71. 2005
- Petschnigg J, Moe OW, Stagljar I (2011) Using yeast as a model to study membrane proteins. Current opinion in nephrology and hypertension 20: 425-432
- Petschnigg J, Wolinski H, Kolb D, Zellnig Gn, Kurat CF, Natter K, Kohlwein SD (2009) Good Fat, Essential Cellular Requirements for Triacylglycerol Synthesis to Maintain Membrane Homeostasis in Yeast. J Biol Chem 284: 30981-30993
- Pineau L, Bonifait L, Berjeaud J-M, Alimardani-Theuil P, Berges T, Ferreira T (2008) A Lipid-mediated Quality Control Process in the Golgi Apparatus in Yeast. Mol Biol Cell 19: 807-821
- Pineau L, Colas J, Dupont S, Beney L, Fleurat-Lessard P, Berjeaud JM, Berges T, Ferreira T (2009) Lipid-Induced ER Stress: Synergistic Effects of Sterols and Saturated Fatty Acids. Trafic
- Pineau L, Ferreira T (2010) Lipid-induced ER stress in yeast and β cells: parallel trails to a common fate. FEMS Yeast Research
- Poon PP, Nothwehr SF, Singer RA, Johnston GC. The Gcs1 and Age2 ArfGAP proteins provide overlapping essential function for transport from the yeast trans-Golgi network. J Cell Biol. 2001 Dec 24;155(7):1239-50
- Robinson M, Poon PP, Schindler C, Murray LE, Kama R, Gabriely G, Singer RA, Spang A, Johnston GC, Gerst JE. The Gcs1 Arf-GAP mediates Snc1,2 v-SNARE retrieval to the Golgi in yeast. Mol Biol Cell. 2006 Apr;17(4):1845-58
- Sampson HM, Robert R, Liao J, Matthes E, Carlile GW, Hanrahan JW, Thomas DY. Identification of a NBD1-binding pharmacological chaperone that corrects the trafficking defect of F508del-CFTR. Chem Biol. Feb 25;18(2):231-42. 2011
- Schneider MF, Marsh D, Jahn W, Kloesgen B, Heimburg T. Network formation of lipid membranes: triggering structural transitions by chain melting. Proc Natl Acad Sci USA. 1999 Dec 7;96(25):14312-7
- Stein DT, Stevenson BE, Chester MW, Basit M, Daniels MB, Turley SD, McGarry JD (1997) The insulinotropic potency of fatty acids is influenced profoundly by their chain length and degree of saturation. The Journal of Clinical Investigation 100: 398-403
- Van Goor F, Straley KS, Cao D, Gonzalez J, Hadida S, Hazlewood A, Joubran J, Knapp T, Makings LR, Miller M, Neuberger T, Olson E, Panchenko V, Rader J, Singh A, Stack JH, Tung R, Grootenhuis PD, Negulescu P. Rescue of DeltaF508-CFTR trafficking and gating in human cystic fibrosis airway primary cultures by small molecules. Am J Physiol Lung Cell Mol Physiol. Jun;290(6):L1117-30. 2006
- Van Goor F, Hadida S, Grootenhuis PD, Burton B, Stack JH, Straley KS, Decker CJ, Miller M, McCartney J, Oison ER, Wine JJ, Frizzell RA, Ashlock M, Negulescu PA. Correction of the F508del-CFTR protein processing defect in vitro by the investigational drug VX-809. Proc Natl Acad Sci USA. Nov 15;108(46):18843-8. 2011
- Wang Y, Bartlett MC, Loo TW, Clarke DM. Specific rescue of cystic fibrosis transmembrane conductance regulator processing mutants using pharmacological chaperones. Mol Pharmacol. Jul;70(1):297-302. 2006
- Wei Y, Wang D, Topczewski F, Pagliassotti MJ. (2006) Saturated fatty acids induce endoplasmic reticulum stress and apoptosis independently of ceramide in liver cells. Vol. 291, pp. E275-281.
- Zhang K, Kaufman RJ. (2006) The unfolded protein response: A stress signaling pathway critical for health and disease. Vol. 66, pp. S102-109.
- Vachel L, Norez C, Becq F, Vandebrouck C. Effect of VX-770 (ivacaftor) and OAG on Ca2+ influx and CFTR activity in G551D and F508del-CFTR expressing cells. J. Cyst. Fibros. 2013 Dec;12(6):584-91.

## Revendications

1. Composé comprenant une tête polaire, comprenant au moins un résidu hydroxyle, sur laquelle est greffé un unique acide gras insaturé comprenant entre 16 et 24 atomes de carbone et ayant 1 à 6 insaturation(s) en configuration *cis* pour une utilisation pour prévenir et/ou traiter, chez un sujet, une lipointoxication par hypoxie liée à la présence en excès dans les membranes biologiques de cellules non adipocytaires, d'acides gras, en particulier d'acides gras à longues chaines saturées, et/ou de stérols ;
ledit composé étant choisi parmi le mannide monooléate, le 3-hydroxy-2,2-bis(hydroxymethyl)propyl oléate, le N,N-diethanololeamide, et leurs mélanges ;
ladite lipointoxication par hypoxie étant associée chez ledit sujet à une pathologie pulmonaire.

2. Composé pour utilisation selon la revendication 1, **caractérisé en ce que** la lipointoxication par hypoxie est à l'origine du dysfonctionnement ou de l'apoptose desdites cellules non adipocytaires par diminution ou suppression de la fluidité de leur membrane plasmique et/ou de la membrane de leurs organelles.

3. Composé pour utilisation selon l'une des revendications précédentes, **caractérisé en ce qu'**il est non toxique pour les cellules capables de synthétiser des lipides neutres, typiquement des triglycérides et/ou stérols estérifiés, en particulier pour les cellules épithéliales bronchiques.

4. Composé pour utilisation selon l'une des revendications 1 à 3, **caractérisé en ce qu'**il est choisi parmi le N,N-diethanololeamide ; alternativement le mannide monooléate, le 3-hydroxy-2,2-bis (hydromethyl)propyl oléate, et leurs mélanges.

5. Composé pour utilisation selon l'une des revendications précédentes, **caractérisé en ce qu'**en restaurant la fluidité des membranes biologique, il prévient et/ou traite la lipointoxication par hypoxie.

6. Composé pour utilisation selon l'une des revendications précédentes, **caractérisé en ce que** (i) il est capable de restaurer la croissance d'un mutant *hem1Δ* de levure *Saccharomyces cerevisiae* lipointoxiqué, (ii) il est capable de réduire ou supprimer la réponse UPR (« *Unfolded Protein Response* »), (iii) il n'est pas toxique pour un mutant QM de levure *Saccharomyces cerevisiae,* et/ou (iv) il réduit ou supprime la mort cellulaire par apoptose d'une cellule de mammifère lipointoxiquée.

7. Composé pour utilisation selon l'une des revendications précédentes, **caractérisé en ce que** la pathologie pulmonaire est une pathologie pulmonaire entrainant une insuffisance respiratoire, plus particulièrement la mucoviscidose ou une broncho-pneumopathie chronique obstructive.

8. Composé pour utilisation selon l'une des revendications précédentes, où le sujet est un animal, typiquement un mammifère, de préférence un être humain.

## Patentansprüche

1. Verbindung umfassend einen polaren Kopf umfassend mindestens einen Hydroxylrest, auf den eine einzige ungesättigte Fettsäure, die zwischen 16 und 24 Kohlenstoffatomen und 1 bis 6 Unsättigung(en) in cis-Konfiguration umfasst, aufgepfropft ist, für eine Anwendung zur Vorbeugung und/oder Behandlung einer hypoxiebedingten Lipidvergiftung bei einem Individuum, die mit dem Vorliegen eines Überschusses an Fettsäuren, insbesondere langkettigten gesättigten Fettsäuren, und/oder Sterolen in biologischen Membranen von Nicht-Adipozyten-Zellen verbunden ist;
wobei die Verbindung aus Mannidmonooleat, 3-Hydroxy-2,2-bis(hydroxymethyl)propyloleat, N,N-Diethanololeamid und ihren Mischungen ausgewählt ist;
wobei die hypoxiebedingte Lipidvergiftung bei dem Individuum mit einer Lungenpathologie assoziiert ist.

2. Verbindung zur Verwendung nach Anspruch 1, **dadurch gekennzeichnet, dass** die hypoxiebedingte Lipidvergiftung auf einer Dysfunktion oder Apoptose der Nicht-Adipozyten-Zellen durch Verringerung oder Suppression der Fluidität ihrer Plasmamembran und/oder der Membran ihrer Organellen beruht.

3. Verbindung zur Verwendung nach einem der vorhergehenden Ansprüche, **dadurch gekennzeichnet, dass** sie für Zellen, die zur Synthese von Neutrallipiden, typischerweise Triglyceriden und/oder veresterten Sterolen, befähigt sind, insbesondere für die Bronchialepithelzellen, nicht toxisch ist.

4. Verbindung zur Verwendung nach einem der Ansprüche 1 bis 3, **dadurch gekennzeichnet**, das sie aus N,N-Diethanololeamid; alternativ dazu Mannidmonooleat, 3-Hydroxy-2,2-bis-(hydromethyl)propyloleat und ihren Mischungen ausgewählt ist.

5. Verbindung zur Verwendung nach einem der vorhergehenden Ansprüche, **dadurch gekennzeichnet, dass** sie durch Wiederherstellen der Fluidität von biologischen Membranen der hypoxiebedingten Lipidvergiftung vorbeugt und/oder diese behandelt.

6. Verbindung zur Verwendung nach einem der vorhergehenden Ansprüche, **dadurch gekennzeichnet, dass** (i) sie fähig ist, das Wachstum einer *hem1Δ-*Mutante der Hefe *Saccharomyces cerevisiae* mit Lipidvergiftung wiederherzustellen, (ii) sie fähig ist, die UPR (*"Unfolded Protein Response"*) zu vermindern oder zu unterdrücken, (iii) sie für eine QM-Mutante der Hefe *Saccharomyces cerevisiae* nicht toxisch ist und/oder (iv) sie den apoptotischen Zelltod einer Säugerzelle mit Lipidvergiftung vermindert oder unterdrückt.

7. Verbindung zur Verwendung nach einem der vorhergehenden Ansprüche, **dadurch gekennzeichnet, dass** es sich bei der Lungenpathologie um eine Lungenpathologie, mit der eine Ateminsuffizienz einhergeht, insbesondere um Mukoviszidose oder eine chronisch-obstruktive Bronchopneumopathie, handelt.

8. Verbindung zur Verwendung nach einem der vorhergehenden Ansprüche, wobei es sich bei dem Individuum um ein Tier, typischerweise einen Säuger, vorzugsweise einen Menschen, handelt.

## Claims

1. Compound comprising a polar head, comprising at least one hydroxyl residue, on which there is grafted a unique unsaturated fatty acid comprising between 16 and 24 carbon atoms and having 1 to 6 unsaturation(s) in *cis* configuration for use for the prevention and/or the treatment of a subject, of the lipotoxicity by hypoxia related to the presence, in excess, in the biological membranes of non-adipocyte cells, of fatty acids, especially saturated long-chain fatty acids and sterols;
said compound is chosen from among mannide monooleate, le 3-hydroxy-2,2-bis (hydromethyl)propyl oleate, N,N-diethanololeamide, and their mixtures;
said lipotoxicity by hypoxia is associated in said subject with a pulmonary pathology.

2. Compound for use according to claim 1, **characterized in that** the lipotoxicity by hypoxia is at the source of dysfunction and/or the apoptosis of said non-adipocyte cells by reducing or even eliminating the fluidity of their plasma membrane and/or the membrane of their organelles.

3. Compound for use according to any one of the preceding claims, **characterized in that** it is non-toxic for cells capable of synthesizing neutral lipids, typically triglycerides and/or esterified sterols, especially for bronchial epithelial cells.

4. Compound for use according to any one of claims 1 to 3, **characterized in that** it is chosen from among N,N-diethanololeamide; alternatively mannide monooleate, 3-hydroxy-2,2-bis (hydromethyl)propyl oleate, and their mixtures.

5. Compound for use according to one of the preceding claims, **characterized in that**, by restoring the fluidity of the biological membranes, it prevents and/or treats lipotoxicity by hypoxia.

6. Compound for use according to one of the preceding claims, **characterized in that** (i) it is capable of restoring the growth of a *hem1Δ* mutant of a lipo-intoxicated yeast *Saccharomyces cerevisiae,* (ii) it is capable of reducing or eliminating the UPR (unfolded protein response), (iii) it is not toxic for a QM mutant of yeast *Saccharomyces cerevisiae,* and/or (iv) it reduces or eliminates cell death by apoptosis of lipo-intoxicated mammal cells.

7. Compound for use according to any one of the preceding claims, **characterized in that** the pulmonary pathology is a pathology leading to a respiratory failure, more particularly cystic fibrosis or a chronic obstructive pulmonary disease.

8. Compound for use according to any one of the preceding claims, where the subject is an animal, typically a mammal, preferably a human being.
